# FASCICULE DE BREVET EUROPEEN

(11) **EP 1 493 035 B1**
(45) Date de publication et mention de la délivrance du brevet: **26.11.2008**
(21) Numéro de dépôt: 03745845.2
(22) Date de dépôt: 10.04.2003
(51) Int. Cl.: G01N 33/68, C12N 9/00, C12N 15/62, C12N 9/12, G01N 33/542, C12Q 1/48, G01N 33/50

(54) **PROCEDE DE SELECTION D'AGENTS BIO-ACTIFS PAR DETECTION D'UNE VARIATION INDUITE DE LA CONCENTRATION INTRACELLULAIRE D'AMPc D'UNE CELLULE VIVANTE SENSIBLE**
VERFAHREN ZUR AUSWAHL VON BIOLOGISCH AKTIVEN MITTELN DURCH NACHWEIS EINER KONZENTRATIONSÄNDERUNG VON cAMP IN EINER LEBENDEN EMPFINDLICHEN ZELLE
METHOD FOR SELECTING BIOACTIVE AGENTS BY DETECTING VARIATION INDUCED IN CYCLIC AMP INTRACELLULAR CONCENTRATION OF A SENSITIVE LIVING CELL

(30) Priorité: 11.04.2002 FR 0204537
(43) Date de publication de la demande: 05.01.2005
(73) Titulaire: Novaleads, 31520 Ramonville Saint Agne (FR)
(72) Inventeur: FURGER, Christophe, F-31000 Toulouse (FR); Lorenzo, Corinne, F-31520 Ramonville Saint Agne (FR)
(74) Mandataire: Cabinet BARRE LAFORGUE & associés
(86) Numéro de dépôt international: PCT/FR2003/001145
(87) Numéro de publication internationale: WO 2003/085405

(56) Documents cités:
- ZACCOLO MANUELA ET AL: "A genetically encoded, fluorescent indicator for cyclic AMP in living cells." NATURE CELL BIOLOGY, vol. 2, no. 1, janvier 2000 (2000-01), pages 25-29, XP002251301 ISSN: 1465-7392 cité dans la demande
- ADAMS S ET AL: "Fluorescence ratio imaging of cyclic AMP in single cells" NATURE, MACMILLAN JOURNALS LTD. LONDON, GB, vol. 349, no. 6311, 21 février 1991 (1991-02-21), pages 694-697, XP002156501 ISSN: 0028-0836 cité dans la demande

## Description

L'invention concerne un procédé de sélection d'agents biologiquement actifs dont l'activité se traduit par une modulation de la voie de transduction du signal AMPc/PKA. L'invention s'étend à un système cellulaire vivant -notamment à l'exception d'un embryon humain, d'un corps humain et d'un élément non isolé d'un corps humain-, adapté à la mise en oeuvre d'un tel procédé.

Dans l'industrie pharmaceutique, le développement d'un nouveau médicament passe par une étape clé : la recherche d'agents biologiquement actifs. Ces agents sont recherchés pour leur capacité à interagir et à activer des cibles cellulaires impliquées dans de nombreuses fonctions physiologiques de l'organisme.

Il existe actuellement de nombreuses banques d'agents potentiellement bio-actifs (molécules générées par la chimie combinatoire ou parallèle, substances d'origine naturelle...) privées ou publiques comprenant un très grand nombre d'agents dont l'activité biologique vis-à-vis de cellules sensibles doit pouvoir être testée.

Au niveau cellulaire, lorsqu'une cible de type récepteur membranaire est activée, une cascade de réactions intracellulaires, appelée transduction du signal, est induite. L'information reçue par le récepteur est alors transférée de la membrane plasmique vers le cytoplasme suivant une voie de transduction du signal particulière dont la nature peut varier suivant celle du récepteur, celle de la cellule et/ou celle du stimulus appliqué sur la cellule.

Parmi les différentes voies de transduction du signal, la voie de transduction de l'AMPc/PKA est une des cascades de signalisation intracellulaires majeures.

Typiquement, la transduction du signal AMPc/PKA débute lorsqu'un ligand se lie à un récepteur couplé aux protéines G (RCPG) et favorise son isomérisation vers une conformation active. Dans cet état actif le récepteur peut interagir et stimuler une protéine G. Deux de ces protéines, appelées Gs et Gi ("s" pour stimulateur et "i" pour inhibiteur), modulent le taux de synthèse de l'AMPc (AMP cyclique ou 3',5'-adénosine monophosphate) en stimulant ou en inhibant l'activité d'une enzyme membranaire, l'adénylyl cyclase. La concentration intracellulaire en AMPc est également dépendante de son taux de dégradation régulé par des enzymes nommées phosphodiestérases. L'augmentation de la concentration intracellulaire de l'AMPc conduit à l'activation de différentes cibles, appelées effecteurs, qui sont soit spécifiques de l'AMPc, telles que la protéine kinase A (PKA) et l'échangeur protéique activé directement par l'AMPc (Epac) ou sélective dans le cas du canal ionique dont l'activité dépend de nucléotides cycliques ("CNG channel"). En activant ces cibles, l'AMPc modifie certaines propriétés de réponses cellulaires très variées comme le potentiel de membrane et la prolifération ou la différenciation cellulaires. La voie AMPc/PKA est une voie de transduction intracellulaire majeure impliquée dans plus de 30% des cibles des agents bio-actifs.

La détection ou le dosage de l'activité de la voie de transduction AMPc/PKA étant donc nécessaire pour le criblage d'agents bio-actifs, plusieurs méthodes reposant sur les propriétés structurelles et fonctionnelles de la PKA, cible spécifique de l'AMPc, ont été proposées.

La PKA est une holoenzyme tétramérique, constituée d'une sous-unité régulatrice dimérique et de deux sous-unités catalytiques monomériques. Dans des conditions de concentration intracellulaire d'AMPc basale, la fonction de la sous-unité régulatrice est de lier la sous-unité catalytique afin de l'inactiver. En revanche, lorsque la concentration intracellulaire de l'AMPc augmente, la sous-unité régulatrice joue le rôle de récepteur intracellulaire de l'AMPc. La liaison de ce dernier sur la sous-unité régulatrice entraîne le changement de conformation de cette dernière permettant la dissociation des sous-unités catalytiques.

Une méthode proposée pour détecter le changement d'état de la PKA, exploite le principe de "transfert d'énergie de fluorescence par résonance" ou FRET. Ce principe repose sur un processus physique, dépendant de la distance, par lequel l'énergie est transmise de manière non radiative d'un chromophore excité, le donneur, à un autre chromophore, l'accepteur, par interaction dipôle-dipôle.

Une première méthode proposée utilisant le FRET (Adams S.R. et al., 1991, Nature, 349, 694-697) consiste à micro-injecter, dans les cellules, des sous-unités catalytiques de la PKA marquées avec la fluorescéine et les sous-unités régulatrices marquées avec la rhodamine. L'activation de la PKA conduit à la dissociation des différentes sous-unités, éliminant ainsi le transfert d'énergie, et donc le phénomène de FRET.

La micro-injection des sous-unités dans les cellules, étape hautement invasive pour ces dernières, délicate, longue et coûteuse à réaliser, est un inconvénient majeur de cette technique.

Une deuxième méthode, inspirée de la précédente, (Zaccolo M. et al., 2000, Nature Cell Biology, 2, 25-29) a alors été proposée pour pallier les problèmes de purification et de micro-injection des sous-unités de la PKA. Cette méthode consiste à produire des cellules transfectées exprimant deux protéines de fusion : une sous-unité régulatrice couplée à une protéine fluorescente, et une sous-unité catalytique couplée à une protéine fluorescente photocomplémentaire de la précédente.

Toujours en utilisant la propriété de FRET, WO 00/49183 et WO 00/75332, proposent de ne faire exprimer qu'une protéine recombinée, la sous-unité régulatrice de la PKA. Celle-ci est marquée au moyen de deux protéines fluorescentes complémentaires, présentes de part et d'autre du domaine de fixation de l'AMPc. La fixation de l'AMPc sur la sous-unité régulatrice modifie la conformation de celle-ci et donc la distance séparant les deux protéines fluorescentes complémentaires, induisant ainsi une variation de FRET.

Les tests précédents utilisant le phénomène de FRET, basés sur la propriété fonctionnelle et structurelle de la PKA ne sont pas réellement utilisables en pratique, sauf à mettre en oeuvre des moyens d'analyse d'images lourds et coûteux et fournissant des résultats d'une fiabilité imparfaite (car liés à une interprétation subjective). En effet, la modification d'intensité de fluorescence reflète des modulations de distance entre les groupements fluorescents complémentaires pouvant être très faibles, donc très difficiles à détecter et surtout impossibles à analyser. Ceci est d'autant plus vrai pour les tests proposés par WO 00/49183 et WO 00/75332.

Une autre méthode proposée pour détecter l'activité de la PKA vise à exploiter le phénomène de redistribution (réarrangement spatial) de divers composants protéiques au sein de la cellule en fonction du stimulus appliqué sur celle-ci. Il est ainsi possible, en suivant la distribution au sein de la cellule d'une protéine donnée, de connaître l'état d'activation de celle-ci. Ainsi, WO 98/45704 propose une méthode de criblage d'agents bio-actifs susceptibles de provoquer l'activation d'une protéine bien définie en fabriquant un système cellulaire dans lequel ladite protéine est fusionnée à une protéine fluorescente de type GFP. Les agents bio-actifs capables d'induire l'activité de ces protéines, engendrent au contact de telles cellules transfectées une redistribution de la fluorescence, redistribution observable en microscopie de fluorescence.

WO 98/45704 propose également d'appliquer cette méthode à la PKA. Pour cela, des cellules exprimant une sous-unité catalytique fluorescente de la PKA ont été proposées. A faible concentration d'AMPc intracellulaire, la fluorescence dans le cytoplasme de ces cellules serait plus ou moins organisée en agrégats, alors qu'à forte concentration d'AMPc, elle serait davantage dispersée au sein du cytoplasme.

L'AMPc est produit par l'adénylyl cyclase au niveau de la membrane plasmique puis diffuse dans le cytoplasme où il rencontre soit la PKA, sa cible, soit une phosphodiestérase qui le rend inactif par dégradation. Il existe donc un gradient négatif de concentration en AMPc à partir de son site de synthèse membranaire. Or, WO 98/45704 détecte des variations de la fluorescence de la PKA présente dans le cytoplasme, donc nécessairement à distance importante de la source de synthèse d'AMPc. Il s'ensuit que cette méthode de détection reste peu sensible en pratique, en particulier pour le criblage de ligands extracellulaires. Ce type de test se trouve donc très limité dans ses applications.

La présente invention se propose de fournir un procédé de sélection d'agents bio-actifs -notamment mais non exclusivement d'agents pharmacologiquement actifs pour l'homme ou l'animal- dont l'activité se manifeste vis-à-vis d'une cellule sensible, par une variation intracellulaire d'AMPc. L'invention vise aussi à proposer un procédé de sélection adapté aux besoins d'un criblage sensible et à haut débit, c'est-à-dire capable de fournir rapidement des résultats sensibles, objectifs, fiables, d'analyse facile et rapide.

L'invention s'étend à un système cellulaire adapté à la mise en oeuvre d'un tel procédé de sélection ainsi qu'à un procédé d'obtention d'un tel système cellulaire.

L'invention s'étend également aux différents "outils" génétiques -notamment séquences d'ADN et vecteurs nucléiques- permettant la réalisation d'un tel système cellulaire.

L'invention concerne donc un procédé de sélection d'agents, dits agents à tester, susceptibles d'être biologiquement actifs vis-à-vis d'un système cellulaire vivant en induisant ou en inhibant une synthèse intracellulaire d'AMPc lorsqu'ils sont en présence d'au moins une cellule sensible d'un système cellulaire vivant. Dans un procédé conforme à la revendication 1 :
- on prépare un système cellulaire comprenant au moins une cellule vivante sensible exprimant au moins une protéine recombinée multimérique spécifique de la voie de transduction AMPc/PKA,
   - ladite protéine recombinée étant, en l'absence d'une synthèse induite d'AMPc, dans un premier état, dit état complexé, où elle forme un complexe, dit complexe RC, comprenant au moins une unité R comportant au moins un site de liaison de l'AMPc, et au moins une unité C, distincte d'une unité R,
   - ladite protéine recombinée étant, en présence d'une synthèse induite d'AMPc, dans un deuxième état, dit état dissocié, où les unités R et C sont dissociées et où au moins une unité R fixe l'AMPc,
- on met en présence ledit système cellulaire avec une quantité d'au moins un agent à tester,
- on détecte l'état, complexé ou dissocié, de ladite protéine recombinée,
caractérisé en ce que :
- au moins l'une des unités, dite unité de translocation, présente une séquence protéique comprenant une séquence d'adressage membranaire présentant un motif de modification post-traductionnel CAAX fonctionnel, c'est-à-dire adapté pour induire une translocation membranaire d'au moins une partie de ladite unité de translocation ; la position de ladite séquence d'adressage membranaire dans ladite séquence protéique étant adaptée pour préserver l'aptitude de la protéine recombinée à former à la membrane un complexe RC en l'absence de synthèse induite d'AMPc, et à se dissocier en présence de synthèse induite d'AMPc,
- au moins une autre unité, dite unité marquée, distincte d'une unité de translocation, présente un groupement, dit groupement de détection, adapté pour :
   - préserver l'aptitude de la protéine recombinée à former un complexe RC en l'absence de synthèse induite d'AMPc, et à se dissocier en présence de synthèse induite d'AMPc, et
   - permettre la détection de l'état de la protéine recombinée à l'aide d'un signal de détection qui, à la membrane, est sensiblement différent selon l'absence ou la présence de synthèse induite d'AMPc.

La présente invention propose ainsi de forcer le recrutement de la PKA à la membrane plasmique afin de :
- rapprocher la PKA de la source de synthèse membranaire de l'AMPc et par conséquent augmenter la sensibilité de la détection de l'AMPc,
- localiser la PKA dans un compartiment intracellulaire (la membrane plasmique) favorable pour une observation fiable, simple, rapide.

L'invention concerne aussi un système cellulaire vivant -notamment à l'exception d'un embryon humain, d'un corps humain ou d'un élément non isolé du corps humain- conforme à la revendication 15, contenant au moins une cellule vivante exprimant au moins une protéine recombinée multimérique spécifique de la voie de transduction AMPc/PKA, telle que mentionnée ci-dessus.

Selon l'invention, pour forcer la localisation de ladite protéine recombinée multimérique spécifique de la voie de transduction AMPc/PKA à la membrane plasmique, le cadre de lecture du gène codant, soit pour une unité R soit pour une unité C, a été fusionné avec une séquence d'adressage membranaire contenant un motif CAAX (où C désigne un résidu cystéine, A un résidu d'acide aminé aliphatique et X un résidu d'acide aminé ne possédant pas de charge).

Le terme consacré de "séquence d'adressage membranaire" désigne classiquement une séquence protéique comprenant ledit motif de modification post-traductionnelle CAAX et une séquence d'une quinzaine d'acides aminés.

Initialement ces séquences d'adressage membranaire ont été mises en évidence chez les protéines de la famille Ras. Les protéines Ras sont des petites protéines G monomériques localisées à la membrane plasmique. Celles-ci jouent un rôle clé dans les systèmes de transduction du signal intracellulaire impliqués dans la régulation de la prolifération et de la différenciation cellulaire.

Dans la cellule, le motif CAAX d'une séquence d'adressage membranaire est reconnu par une enzyme, de la famille des isoprényl-transférases, conduisant à la farnésylation ou la géranylgéranylation du résidu cystéine puis au clivage protéolytique des résidus AAX terminaux. L'ensemble de ces modifications est nécessaire pour l'ancrage des protéines Ras à la membrane plasmique. Aussi, on dit d'un motif de modification post-traductionnelle CAAX d'une séquence protéique, qu'il est fonctionnel lorsqu'il peut conduire à l'encrage à la membrane d'au moins une partie de ladite séquence protéique lorsque celle-ci est exprimée dans une cellule.

A titre d'exemples non limitatifs de séquences d'adressage membranaire contenant un motif de modification post-traductionnelle CAAX pouvant être utilisées dans le cadre de la présente invention, on peut tout d'abord citer les trois gènes *ras* découverts chez l'homme (Lowy D.R. et Willumsen B.M., 1993, Ann. Rev. Bioch., 62, 851-891) : H-Ras (Harvey), K-Ras (Kirsten) et N-Ras (*neuroblastoma*). L'expression du gène K-Ras peut conduire par épissage alternatif des exons 4A et 4B à la production de deux protéines nommées K-Ras 4A et K-Ras 4B. Toutes les protéines Ras possèdent dans leur partie carboxyl-terminale une séquence d'adressage à la membrane plasmique d'une vingtaine d'acides aminés incluant un enchaînement de quatre acides aminés C-A-A-X.

Avantageusement, une séquence d'adressage membranaire conforme à l'invention peut correspondre aux extrémités carboxyl-terminales de la protéine H-Ras, telle que la séquence KLNPPDESGPGCMSCKCVLS (SEQ ID N° 1) ; de la protéine K-Ras 4A, telle que la séquence KISKEEKTPGCVKIKKCIIM (SEQ ID N° 2) ; de la protéine K-Ras 4B, telle que la séquence MSKDGKKKKKKSKTKCVIM (SEQ ID N° 3) ; de la protéine N-Ras, telle que la séquence KLNSSDDGTQGCMGLPCVVM (SEQ ID N° 4).

D'autres séquences d'adressage membranaire peuvent être utilisées dans le cadre de la présente invention, par exemple, des séquences comprenant les séquences précédemment citées ou des séquences provenant de l'extrémité carboxyl-terminale de protéines Ras, autres que des protéines Ras humaines, notamment de rat, de souris, de poulet, de xénope, de cochon, de drosophile, de nématode, de neurospora, de levure, de champignon, de poisson rouge (Lowy D.R. et Willumsen B.M., 1993, Ann. Rev. Bioch., 62, 851-991).

Avantageusement et selon l'invention, le groupement de détection de l'unité marquée est un groupement luminophore choisi parmi :
- une protéine fluorescente naturelle ou une protéine fluorescente mutée, par addition, délétion ou substitution d'un ou plusieurs acides aminés d'une protéine naturelle, cette protéine fluorescente mutée conservant la propriété de fluorescence,
- un fragment fluorescent issu d'une protéine fluorescente naturelle ou d'une protéine fluorescente mutée adapté pour présenter la propriété de fluorescence de la protéine fluorescente dont il est issu,
- une protéine bioluminescente naturelle -notamment de type luciférase- ou une protéine bioluminescente mutée, par addition, délétion ou substitution d'un ou plusieurs acides aminés d'une protéine naturelle, cette protéine bioluminescente mutée conservant la propriété de bioluminescence,
- la fluorescéine, la rhodamine, le Bodipy®, la cyanine, le Nile Red@, Texas Red@, les chélates de terres rares, les indo- et carbocyanines, le diphénylhéxatriène, les dérivés aromatiques luminescents.

Avantageusement et selon l'invention, le groupement protéique luminophore est la Cycle3-GFP, un mutant thermostable de la protéine fluorescente verte (GFP) de la méduse *Aequorea victoria.* Il s'agit d'une protéine de 238 acides aminés qui peut être exprimée dans des cellules eucaryotes en conservant toutes ses propriétés de fluorescence. L'excitation de la Cycle3-GFP se produit entre 350 nm et 490 nm avec un maximum d'absorption à 398 nm et son émission de fluorescence se produit avec un maximum à 508 nm. Une protéine recombinée multimérique selon l'invention, en l'absence de synthèse induite d'AMPc, se trouve donc dans un état complexé, localisée à la membrane plasmique de façon constitutive par l'intermédiaire d'une unité de translocation selon l'invention. La dissociation de ce complexe provoquée lors d'une synthèse induite d'AMPc peut être suivie sur des cellules vivantes grâce à la propriété de fluorescence de la Cycle3-GFP de l'unité marquée. En terme de fluorescence, l'augmentation du taux intracellulaire d'AMPc se traduit par un déplacement de la fluorescence de la membrane plasmique vers le cytoplasme. De façon réciproque, ce procédé permet également l'observation d'une diminution du taux intracellulaire d'AMPc qui se traduit alors par un déplacement de la fluorescence du cytoplasme vers la membrane plasmique.

La distinction entre le marquage par luminescence d'une membrane et celui du cytoplasme est suffisamment prononcée pour permettre une détection objective et fiable de l'état de la protéine par une simple observation en microscopie de fluorescence.

Avantageusement et selon l'invention, l'unité de translocation est l'unité R, et l'unité marquée est l'unité C.

Selon une variante préférée de l'invention, la détection de l'état de la protéine recombinée multimérique spécifique de la voie de transduction AMPc/PKA se fait par détection d'une émission ou d'une extinction lumineuse du couplage de luminescence entre deux groupements luminescents complémentaires, en exploitant le principe de FRET. Selon une autre variante, ladite détection peut également se faire en exploitant le principe de BRET (transfert de bioluminescence par résonance).

Pour ce faire, on introduit dans les cellules sensibles au moins un agent, dit agent membranaire de couplage, apte à s'associer à la membrane plasmique, et marqué avec au moins un groupement luminescent, dit deuxième luminophore, distinct du groupement luminescent porté par l'unité marquée, dit premier luminophore. Conformément à cette variante de l'invention, ces deux luminophores sont photocomplémentaires de sorte que l'unité marquée et l'agent membranaire de couplage créent entre eux un phénomène de couplage de luminescence.

Pour la mise en oeuvre de cette variante de réalisation, les inventeurs ont déterminé d'une part, qu'il est possible d'incorporer dans la membrane cellulaire des agents membranaires de couplage, marqué par des groupements luminescents, qui peuvent bénéficier de la dynamique latérale de la membrane cellulaire (en étant eux-même soumis à cette dynamique) pour se répartir de façon homogène au sein de celle-ci et en une quantité qui simultanément soit assez faible pour ne pas affecter les propriétés fonctionnelles de la membrane cellulaire et de la cellule vivante, mais soit assez importante pour assurer la réalisation du couplage de luminescence lorsque l'unité marquée est recrutée à tout endroit de la membrane cellulaire. Les inventeurs ont constaté que grâce au fait que l'invention met à profit la versatilité des composants membranaires décrite dans le modèle des membranes dit "mosaïque fluide", (Singer S.J. et Nicolson G.L., 1972, Science, 175, 720-731), statistiquement, il se trouve toujours un agent membranaire de couplage à proximité de l'unité de translocation, et ainsi donc également à proximité de l'unité marquée lorsque celle-ci est recrutée à la membrane, et à une distance de celle-ci qui en pratique correspond à l'obtention du couplage de luminescence tel que le FRET ou le BRET.

On sait en effet qu'une émission de luminescence spécifique par couplage de luminescence se produit ou est modifiée lorsque :
- (a) deux groupements photocomplémentaires sont proches dans l'espace (notamment de 1 à 10 nm) d'une distance inférieure ou égale à une valeur seuil prédéterminée (2 Ro, où Ro est le rayon de Forster dans le cas du FRET),
- (b) le spectre d'émission de l'un des groupements photocomplémentaires, dit donneur, et le spectre d'absorption de l'autre groupement photocomplémentaire, dit accepteur, se recouvrent au moins en partie (Lakey et al., 1991, J. Mol. Biol., 1218, 639-653).

Egalement, les inventeurs ont mis en évidence qu'il existe des couples de luminophores compatibles avec les agents membranaires (phospholipides). En particulier, on a trouvé des lipides pouvant porter un composé fluorescent compatible avec les membranes cellulaires naturelles (pouvant faire office d'agents membranaires de couplage) et réalisant un couplage de fluorescence par FRET avec une GFP et notamment avec la GFP naturelle.

Avantageusement et selon l'invention, les premier et deuxième luminophores sont choisis pour réaliser un couplage de luminescence par transfert d'énergie par résonance, notamment par FRET.

Avantageusement et selon l'invention, l'un au moins des premier et deuxième luminophores est choisi parmi :
- une molécule susceptible d'absorber la lumière émise par une protéine fluorescente,
- une protéine fluorescente naturelle ou une protéine fluorescente mutée, par addition, délétion ou substitution d'un ou plusieurs acides aminés d'une protéine fluorescente naturelle, cette protéine fluorescente mutée conservant la propriété de fluorescence,
- un fragment fluorescent issu d'une protéine fluorescente naturelle ou d'une protéine fluorescente mutée adapté pour présenter la propriété de fluorescence de la protéine fluorescente dont il est issu,
- une protéine bioluminescente naturelle -notamment de type luciférase- ou une protéine bioluminescente mutée, par addition, délétion ou substitution d'un ou plusieurs acides aminés d'une protéine naturelle, cette protéine bioluminescente mutée conservant la propriété de bioluminescence,
- la fluorescéine, la rhodamine, le Bodipy®, la cyanine, le Nile Red®, le Texas Red®, les chélates de terres rares, les indo- et carbocyanines, le diphénylhexatriène, les dérivés aromatiques luminescents.

Avantageusement et selon l'invention, l'un au moins des premier et deuxième luminophores est une protéine autofluorescente naturelle de cnidaire, notamment la protéine fluorescente verte GFP de la méduse *Aequorea victoria.*

Avantageusement et selon l'invention, on utilise un agent membranaire de couplage comprenant un ligand lipophile qui peut être marqué par le deuxième luminophore. A titre de ligands lipophiles, on peut citer les phospholipides, les acides gras, les glycérides, le cholestérol, les céramides, les amines grasses, les agents luminophores lipophiles ou les agents dérivés de ces composés. Un agent luminophore lipophile peut constituer à lui seul, un agent membranaire de couplage.

Avantageusement et selon l'invention, ladite protéine recombinée multimérique spécifique de la voie de transduction AMPc/PKA est une protéine de type PKA recombinée ; les unités R et C correspondent respectivement aux sous-unités régulatrice et catalytique de la PKA, recombinées.

On notera que les séquences des sous-unités régulatrices et catalytiques peuvent être des séquences aussi bien homologues qu'orthologues de la PKA, c'est-à-dire des séquences qui proviennent ou non d'une même espèce.

Avantageusement et selon l'invention, l'unité de translocation est une unité régulatrice recombinée issue de la fusion par voie génétique :
- d'au moins une partie de la séquence protéique d'une sous-unité régulatrice choisie parmi les isoformes : RIα, RIβ, RIIα, RIIβ,
- et d'une séquence d'adressage membranaire.

Plus particulièrement, le tableau 1 donne quelques exemples, non limitatifs, d'isoformes de sous-unités régulatrices présentes dans l'espèce humaine, mais également dans de nombreuses autres espèces, pouvant être utilisées pour l'obtention d'une unité de translocation selon l'invention.

**TABLEAU 1**

| **ESPECE** | **BASE DE DONNEES** | **TYPE ISOFORME** | **REFERENCES** | |
|---|---|---|---|---|
| HOMO SAPIENS | SWISSPROT | RIα | ID | KAP0_HUMAN |
| | | | AC | P10644 |
| | PIR | RIIα | ID | OKHUR2 |
| | | | AC | SO3885 |
| | PIR | RIβ | ID | OKHUR1 |
| | | | AC | JH0392 |
| | PIR | RIIβ | ID | OKHUR2 |
| | | | AC | A40915 |
| BOVIN | SWISSPROT | RIα | ID | KAP0_BOVIN |
| | | | AC | P00514 |
| | SWISSPROT | RIIα | ID | KAP2_BOVIN |
| | | | AC | P00515 |
| | PIR | RIIβ | ID | OKBOR2 |
| | | | AC | A36528 |
| COCHON | PIR | RIα | ID | OKPG1R |
| | | | AC | S00083 |
| RAT | PIR | RIα | ID | OKRT1R |
| | | | AC | A26910 |
| | SWISSPROT | RIβ | ID | KAP1_RAT |
| | | | AC | P81377 |
| | PIR | RIIα | ID | OKRT2R |
| | | | AC | A28325 |
| | SWISSPROT | RIIβ | ID | KAP3_RAT |
| | | | AC | P12369 |
| SOURIS | PIR | RIIα | ID | OKMS2R |
| | | | AC | B28325 |
| | PIR | RIβ RI | ID | OKMSR1 |
| | | | AC | A30205 |
| DROSOPHILE | SWISSPROT | | ID | KAPR_DROME |
| | | | AC | P16905 |
| | SPTREMBL | RII | ID | Q9NB18 |
| | | | AC | Q9NB18 |
| CHAMPIGNON | SPTREMBL | | ID | Q9C196 |
| Aspergillus niger | | | AC | Q9C196 |
| CHAMPIGNON | GENPEPT | | ID | AF401202_1 |
| Aspergillus fumigatus | | | AC | AF401202 |
| CHAMPIGNON | SPTREMBL | | ID | Q9HEP7 |
| Blumeria graminis | | | AC | Q9HEP7 |
| CHAMPIGNON | SPTREMBL | | ID | Q9XTM6 |
| Euplotes octocarinatus | | | AC | Q9XTM6 |
| CHAMPIGNON | SPTREMBL | | ID | Q9C1C2 |
| Glomerella lagenarium | | | AC | Q9C1C2 |
| CHAMPIGNON | SWISSPROT | | ID | KAPR_BLAEM |
| Blastocladiella emersonii | | | AC | P31320 |
| CHAMPIGNON | SWISSPROT | | ID | KAPR_COLTR |
| Colletotrichum trifolii | | | AC | 042794 |
| CHAMPIGNON | SWISSPROT | | ID | KAPR_EMENI |
| Emericella nidulans | | | AC | 059922 |
| CHAMPIGNON | SWISSPROT | | ID | KAPR_MAGGR |
| Magnaporthe grisea | | | AC | 014448 |
| CHAMPIGNON | SWISSPROT | | ID | KAPR_NEUCR |
| Neurospora crassa | | | AC | Q01386 |
| CHAMPIGNON | SWISSPROT | RII | ID | KAPR_STRPU |
| Strongylocentrotus purpuratus | | | AC | Q26619 |
| CHAMPIGNON | SWISSPROT | | ID | KAPR_USTMA |
| Ustilago maydis | | | AC | P49605 |
| LEVURE | SWISSPROT | | ID | KAPR_YEAST |
| Saccharomyces cerevisiae | | | AC | P07278 |
| CHAMPIGNON | SPTREMBL | | ID | Q9P8K6 |
| Mucor rouxii | | | AC | Q9P8K6 |
| LEVURE | SPTREMBL | | ID | Q9HEW1 |
| Candida albicans | | | AC | Q9HEW1 |
| LEVURE | PIR | | ID | OKBYRC |
| Saccharomyces cerevisiae | | | AC | A25868 |
| Aplysia californica | PIR | RI | ID | OKGAR1 |
| | | | AC | JH0590 |
| Caenorhabditis elegans | PIR | RI | ID | OKKW1R |
| | | | AC | A35076 |

Avantageusement, une unité de translocation conforme à l'invention peut avoir pour séquence protéique SEQ ID N° 5, et être codée par une séquence nucléique telle que SEQ ID N° 6.

Ces différentes isoformes de la PKA forment une famille structurellement et fonctionnellement homogène de protéines.

Bien que codées par des gènes distincts, les sous-unités régulatrices de la PKA sont homologues pour une espèce donnée. Les sous-unités RIα et RIβ comme RIIα et RIIβ présentent entre elles de fortes similarités de séquence en acides aminés respectivement 81% et 68% d'identité. D'un point de vue phylogénétique, les sous-unités régulatrices orthologues sont très conservées et en conséquence présentent, chez les eucaryotes supérieurs, de fortes similarités de séquences en acides aminés de 87% à 98% d'identité (Tasken K.K. et al., 1997, Advances in Second Messenger and Phosphoprotein Research, 31, 191-204).

Les sous-unités régulatrices RIα et RIIα sont exprimées de façon ubiquitaire dans les cellules animales. La proportion relative de chacune peut cependant varier d'un type cellulaire à un autre. L'expression des sous-unités régulatrices RIβ et RIIβ, quant à elle, est plutôt spécifique du type tissulaire. Par exemple, RIIβ est exprimée dans le tissu neuronal, dans les cellules de la granulosa et des testicules, et dans les cellules rénales.

Les sous-unités régulatrices de la PKA (aussi bien d'humain, de rat, de souris, de bovin) comprennent un motif de 17 résidus d'acide aminé : GSFGELAL[IM]Y[GN]TPRAAT (équivalent aux séquences SEQ ID N°7, SEQ ID N°8, SEQ ID N°9, SEQ ID N°10), les crochets signifiant qu'il s'agit de l'un ou l'autre acide aminé.

On note par ailleurs que les isoformes de la PKA forment une famille fonctionnellement homogène et redondante. La preuve de cette redondance repose sur la mise en évidence, dans la plupart des études, de l'aptitude des isoformes de la PKA de se compenser fonctionnellement les unes aux autres lors de la perte de l'une d'entre elles. Par exemple, des études récentes ont montré que lorsqu'un gène codant pour l'une des sous-unités régulatrices était invalidé, le taux d'autres sous-unités régulatrices augmentait de façon à compenser cette perte (Amieux P.S. et al., 1997, The Journal Biological Chemistry, 272, 3993-3998). Cette augmentation peut être observée dans le système nerveux de souris dont le gène codant pour RIβ a été invalidé mais également dans le système nerveux et le tissu adipeux de souris dont RIIβ a été invalidé (Brandon E.P. et al., 1997, Current Opinion in Neurobiology, 7, 397-403).

Les différentes isoformes homologues (intra-espèce) et orthologues (inter-espèce) de la PKA étant fonctionnellement homogènes, on peut donc avantageusement les utiliser indifféremment, pour mettre en oeuvre l'invention.

La région amino-terminale (résidus 1 à 140) des sous-unités régulatrices est riche en résidus hydrophobes et en acides aminés chargés. La fonction majeure de cette région est d'assurer l'interaction entre les deux protomères de la sous-unité régulatrice dimérique. Le domaine de dimérisation est restreint à un petit segment d'au moins 40 acides aminés. Les sous-unités régulatrices possèdent, dans leur moitié amino-terminale, le domaine de liaison à la sous-unité catalytique que l'on qualifie couramment de "hinge region" (région charnière). Cette région est constituée d'une séquence d'acides aminés similaire à celle des substrats de la sous-unité catalytique. Outre le domaine de dimérisation et le domaine d'interaction avec la sous-unité catalytique, les sous-unités régulatrices possèdent dans leur partie carboxyl-terminale deux domaines de liaison de l'AMPc en tandem. Ces deux domaines se distinguent par la cinétique de liaison de l'AMPc. On différencie ainsi le site A, ou site de liaison rapide, et le site B, ou site de liaison lent.

Avantageusement et selon l'invention, l'unité marquée est une séquence protéique comprenant au moins une partie de la séquence d'une sous-unité catalytique choisie parmi les isoformes Cα, Cβ et Cγ, marquée par un groupement de détection.

Plus particulièrement, le tableau 2 donne des exemples d'isoformes de sous-unités catalytiques présentes dans l'espèce humaine, mais également dans d'autres espèces, qui peuvent être utilisées en vue d'obtenir une unité marquée selon l'invention.

**TABLEAU 2**

| **ESPECE** | **BASE DE DONNEES** | **TYPE ISOFORME** | **REFERENCES** | |
|---|---|---|---|---|
| HOMO SAPIENS | PIR | Cα | ID | OKHU2C |
| | | | AC | S01404 |
| | PIR | Cβ | ID | OKHUCB |
| | | | AC | A34724 |
| | SWISSPROT | Cγ | ID | KAPG_HUMAN |
| | | | AC | P22612 |
| MACAQUE | SWISSPROT | Cγ | ID | KAPG_MACMU |
| | | | AC | 062846 |
| BOVIN | PIR | Cα | ID | OKBO2C |
| | | | AC | S27159 |
| | PIR | Cβ1 | ID | OKBOB1 |
| | | | AC | A253345 |
| | SWISSPROT | Cβ2 | ID | KAPI_BOVIN |
| | | | AC | P24256 |
| COCHON | PIR | Cα | ID | S00085 |
| | | | AC | S00085 |
| | SWISSPROT | Cβ | ID | KAPB_PIG |
| | | | AC | P05383 |
| CHIEN | SPTREMBL | | ID | Q16933 |
| | | | AC | Q16933 |
| RAT | PIR | Cα | ID | OKRT2C |
| | | | AC | S16240 |
| | PIR | Cβ | ID | OKRTCB |
| | | | AC | JQ1139 |
| SOURIS | PIR | Cα | ID | KAPA_MOUSE |
| | | | AC | P05132 |
| | PIR | Cβ | ID | KAPB_MOUSE |
| | | | AC | P05206 |
| DROSOPHILE | PIR | | ID | C31751 |
| | | | AC | C31751 |
| HAMSTER | PIR | Cα | ID | OKHYCA |
| | | | AC | B40384 |
| CHAMPIGNON | SPTREMBL | | ID | P87077 |
| Aspergillus niger | | | AC | P87077 |
| CHAMPIGNON | SPTREMBL | | ID | Q9HGU6 |
| Blumeria graminis | | | AC | Q9HGU6 |
| CHAMPIGNON | SPTREMBL | | ID | 097114 |
| Amblyomma americanum | | | AC | 097114 |
| CHAMPIGNON | SPTREMBL | | ID | Q9UUS9 |
| Erysiphe graminis | | | AC | Q9UUS9 |
| CHAMPIGNON | SPTREMBL | | ID | Q12741 |
| Blastocladiella emersonii | | | AC | Q12741 |
| CHAMPIGNON | SPTREMBL | | ID | 042793 |
| Colletotrichum trifolii | | | AC | 042793 |
| CHAMPIGNON | SPTREMBL | | ID | Q9P472 |
| Emericella nidulans | | | AC | Q9P472 |
| CHAMPIGNON | SPTREMBL | | ID | Q9P466 |
| Neurospora crassa | | | AC | Q9P466 |
| LEVURE | SPTREMBL | | ID | Q9HEW0 |
| Candida albicans | | | AC | Q9HEW0 |
| LEVURE | SWISSPROT | | ID | KAPA_YEAST |
| Saccharomyces cerevisiae | | | AC | P06244 |
| | SWISSPROT | | ID | KAPB_YEAST |
| | | | AC | P06245 |
| Aplysia californica | SPTREMBL | | ID | Q16958 |
| | | | AC | Q16958 |
| Dictyostelium discoidum | SWISSPROT | | ID | KAPC_DICDI |
| | | | AC | P34099 |
| Oryctolagus cuniculus | GENPEPT | Cα | ID | AF367428-1 |
| | | | AC | AF367428 |

La forme Cα est exprimée de façon constitutive dans la plupart des cellules, alors que Cβ et Cγ ne sont exprimées que dans certains types de tissus. Des travaux récents ont révélé l'existence de variants d'épissage pour les formes Cα humaine (Cα2), Cβ bovine (Cβ2) et Cβ murine (Cβ2, Cβ3). Toutes les sous-unités catalytiques possèdent un noyau catalytique commun à toutes les protéines kinases. La majorité des sites fonctionnels nécessaires pour la catalyse sont situés dans ce noyau. On trouve dans la partie amino-terminale un site de liaison à l'ATP et dans la partie carboxyl-terminale un site de liaison peptidique. Toutes les sous-unités catalytiques exceptés les variants d'épissage possèdent à leur extrémité amino-terminale un site de myristoylation. Ce site de modification post-traductionnelle semble être impliqué dans la stabilisation de la structure secondaire de la sous-unité catalytique.

Avantageusement, un système cellulaire selon l'invention est obtenu par transfection d'une cellule sensible au moyen de séquences d'ADN codant pour l'unité de translocation et l'unité marquée selon l'invention. Toutefois, en variante, rien n'empêche d'obtenir un tel système en micro-injectant lesdites unités directement dans au moins une cellule sensible.

L'invention s'étend ainsi à un procédé d'obtention d'une cellule sensible transfectée exprimant au moins une protéine recombinée multimérique spécifique de la voie de transduction AMPc/PKA,
- ladite protéine recombinée étant, en l'absence de synthèse induite d'AMPc, dans un premier état, dit état complexé, où il forme un complexe, dit complexe RC, comprenant au moins une unité R comportant au moins un site de liaison de l'AMPc, et au moins une unité C, distincte d'une unité R,
- ladite protéine recombinée étant, en présence de synthèse induite d'AMPc, dans un deuxième état, dit état dissocié, où les unités R et C sont dissociées et où R fixe l'AMPc,
caractérisé en ce qu'il comprend les étapes consistant à :
- transfecter une cellule sensible avec un vecteur dit premier vecteur, contenant une cassette d'expression comprenant une séquence nucléique codant pour l'une des unités, dite unité de translocation, dont la séquence protéique comprend un motif d'adressage membranaire présentant un motif de modification post-traductionnelle CAAX fonctionnel ; la position de ladite séquence d'adressage membranaire dans ladite séquence protéique étant adaptée pour préserver l'aptitude de la protéine recombinée à former à la membrane un complexe RC en l'absence de synthèse induite d'AMPc et à se dissocier en présence de synthèse induite d'AMPc,
- transfecter ladite cellule avec un vecteur, dit deuxième vecteur, contenant une cassette d'expression codant pour une autre unité, dite unité marquée, distincte d'une unité de translocation, et dont la séquence protéique présente un groupement, dit groupement de détection, adapté pour :
   - préserver l'aptitude de la protéine recombinée à former un complexe RC en l'absence de synthèse induite d'AMPc, et à se dissocier en présence de synthèse induite d'AMPc, et
   - permettre la détection de l'état de la protéine recombinée à l'aide d'un signal de détection qui, à la membrane, est sensiblement différent selon l'absence ou la présence de synthèse induite d'AMPc.

Les méthodes permettant l'expression d'un transgène dans une cellule hôte sont aujourd'hui très variées et bien connues en soi (Sambrook J., Fritsch E.F. et Maniatis T. - Molecular Cloning - Cold Spring Harbor Laboratory Press - 1989).

De manière schématique, selon l'invention, chaque séquence nucléique, codant pour l'unité R ou pour l'unité C, est insérée dans une construction nucléique appelée cassette d'expression, dans laquelle ladite séquence nucléique est liée de manière opérante à des éléments permettant son expression -notamment sa régulation-. Parmi ces éléments, on peut citer notamment les promoteurs, les activateurs et les terminateurs de transcription. De nombreuses séquences d'expression -notamment de régulation- sont actuellement connues. Leur choix sera notamment apprécié selon le niveau d'expression souhaité du transgène, et la nature de la cellule à transfecter.

La cassette d'expression est insérée dans un vecteur nucléotidique, tel qu'un plasmide, qui peut en outre comprendre un gène marqueur permettant de sélectionner une cellule transfectée d'une cellule qui ne contient pas l'ADN étranger transfecté.

Les vecteurs ainsi construits sont utilisables pour la transfection, ou plutôt la co-transfection de cellules hôtes, selon toute technique également connue en elle-même.

On peut citer notamment les méthodes de transfert direct de gène par micro-injection, infiltration sous vide, électroporation, choc thermique, bombardement par canon à particules recouvertes de l'ADN plasmidique d'intérêt.

On peut citer aussi des méthodes de transfert indirect, dans lesquelles les premier et deuxième vecteurs sont transférés, préférentiellement séparément dans des parasites cellulaires tels que bactéries et virus susceptibles d'infecter les cellules hôtes en permettant l'intégration de l'ADN étranger dans le génome de ces dernières.

L'invention s'étend également en tant que produit nouveau à une protéine recombinée de séquence protéique SEQ ID N°5 ; ladite protéine recombinée étant susceptible d'être exprimée au niveau de la membrane plasmique d'une cellule, de fixer l'AMPc, de se lier à une sous-unité catalytique de la PKA lorsqu'elle ne fixe pas d'AMPc et de se dissocier de ladite sous-unité catalytique lorsqu'elle fixe l'AMPc.

Avantageusement et selon l'invention, une telle protéine recombinée comprend la séquence protéique SEQ ID N° 5 ou la séquence protéique SEQ ID N° 5 clivée de ces résidus V-L-S carboxyl-terminaux. Une telle protéine peut être codée, de manière non limitative compte tenu de la dégénérescence du code génétique, par une séquence nucléique comprenant la séquence SEQ ID N°6.

L'invention s'étend enfin à une séquence nucléique SEQ ID N°6 codant pour une protéine recombinée selon l'invention.

L'invention concerne aussi un procédé de sélection d'agents bio-actifs, un système cellulaire, un procédé d'obtention d'une cellule sensible exprimant une protéine recombinée multimérique spécifique de la voie de transduction AMPc/PKA, une protéine recombinée et une séquence nucléique codant pour cette dernière, caractérisés, en combinaison, par tout ou partie des caractéristiques ci-dessus ou ci-après.

D'autres buts, caractéristiques et avantages de l'invention apparaîtront à la lecture des exemples suivants qui se réfèrent aux figures annexées, dans lesquelles :
- la figure 1 représente la carte de restriction du vecteur d'expression pet11d/RIIα contenant une séquence codant pour l'isoforme RIIα de souris,
- la figure 2 représente la carte de restriction du vecteur d'expression pet11d/RIIα-CAAX contenant une séquence codant pour la protéine de fusion RIIα-CAAX,
- la figure 3 représente la carte de restriction du vecteur d'expression pcDNA3.1,
- la figure 4 représente la carte de restriction du vecteur d'expression pcDNA3.1/RIIα permettant l'expression de l'isoforme RIIα,
- la figure 5 représente la carte de restriction du vecteur d'expression pcDNA3.1/RIIα-CAAX permettant l'expression de la protéine de fusion RIIα-CAAX,
- la figure 6 représente la carte de restriction du vecteur d'expression pCMV-PKA contenant une séquence codant pour une sous-unité catalytique de la PKA (isoforme Cα),
- la figure 7 représente la carte de restriction du vecteur d'expression pcDNA3.1/NTGFP-K,
- la figure 8 représente la carte de restriction du vecteur d'expression pcDNA3.1/GFP-Cα permettant l'expression de la protéine de fusion GFP-Cα,
- les figures 9a à 9c, 11a à 11d et 12a à 12d sont des vues photographiques en microscopie de fluorescence de cellules HEK 293 transfectées,
- les figures 10a, 10b, 11e, 13a et 13b sont des vues photographiques de membrane de nitrocellulose résultant de la détection protéique par Western Blotting,
- les figures 13a' et 13b' sont des représentations graphiques de l'intensité des détections protéiques obtenues par Western Blotting,
- les figures 14a à 14d, 15a à 15d sont des vues photographiques en microscopie de fluorescence des cellules COS-7 transfectées,
- la figure 16 est une représentation graphique du spectre d'émission de fluorescence des cellules COS-7 transfectées,
- la figure 17a est une représentation graphique de la variation de fluorescence de la GFP-Cα et de la sonde DiI,
- la figure 17b est une représentation graphique de la variation du FRET entre la GFP-Cα et la sonde DiI, après induction de la translocation de la protéine.

### EXEMPLE 1 : Clonage

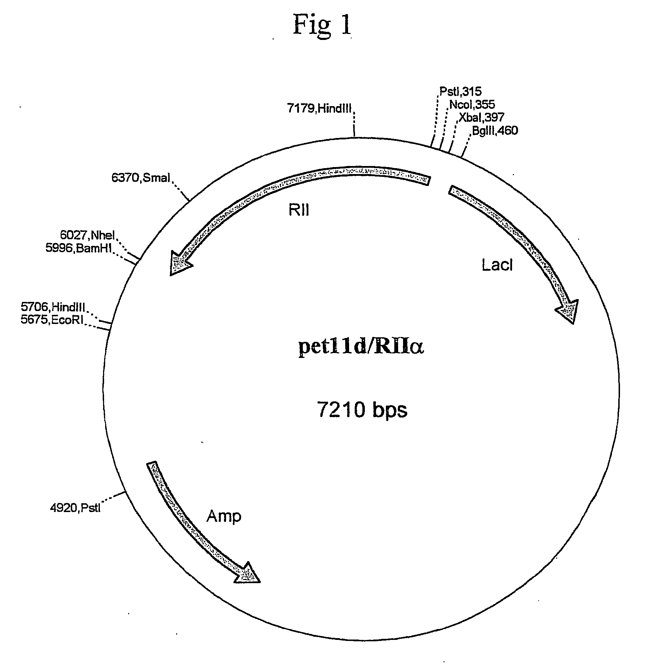

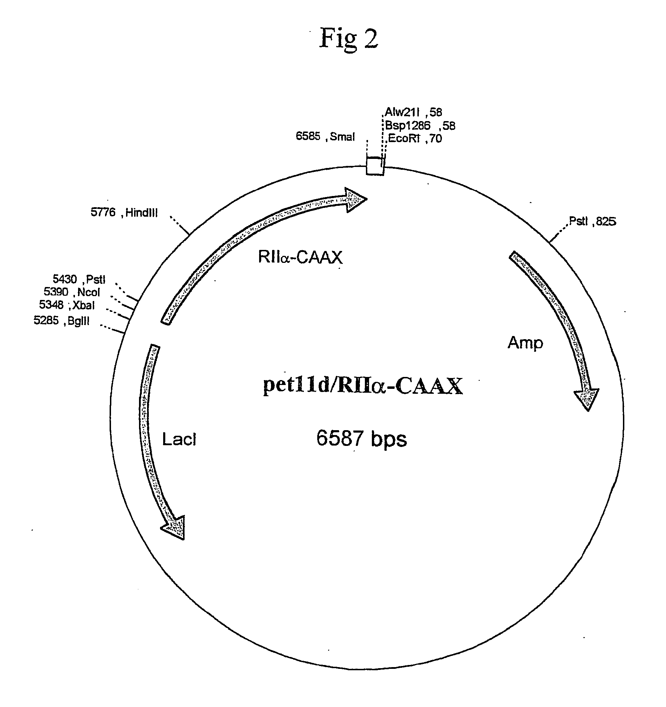

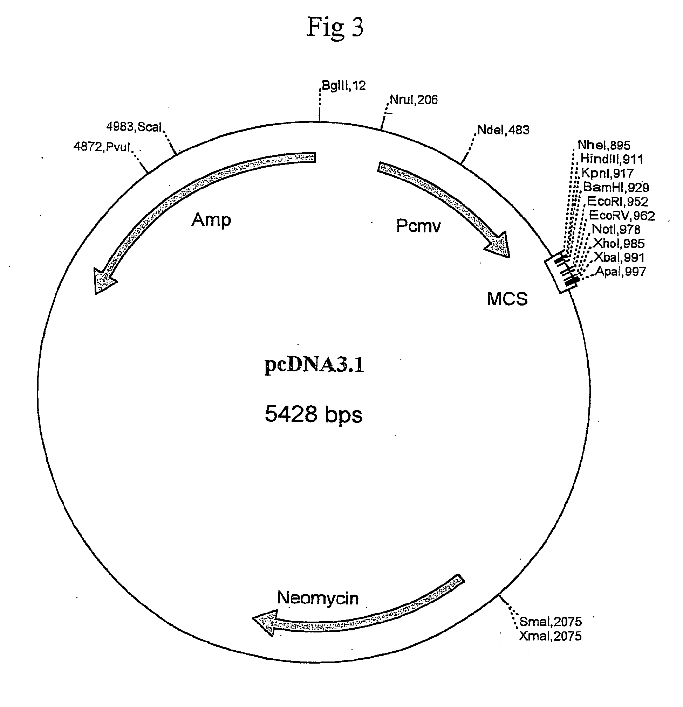

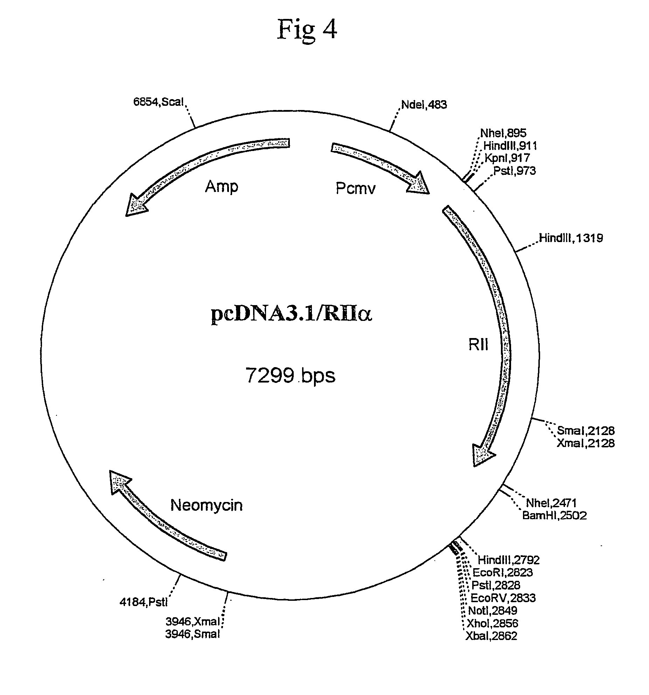

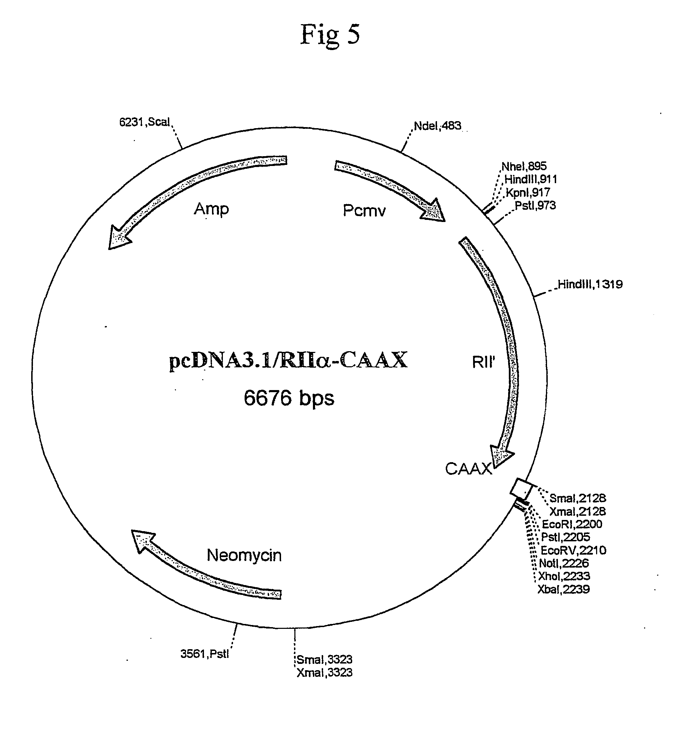

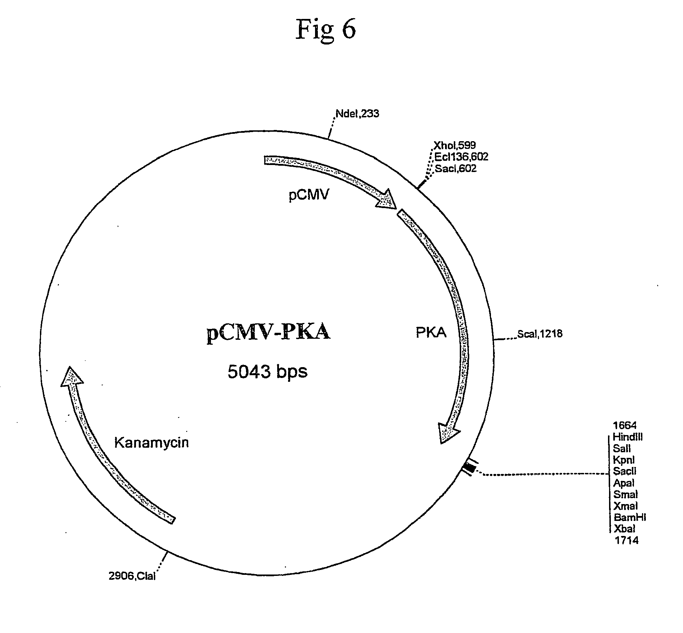

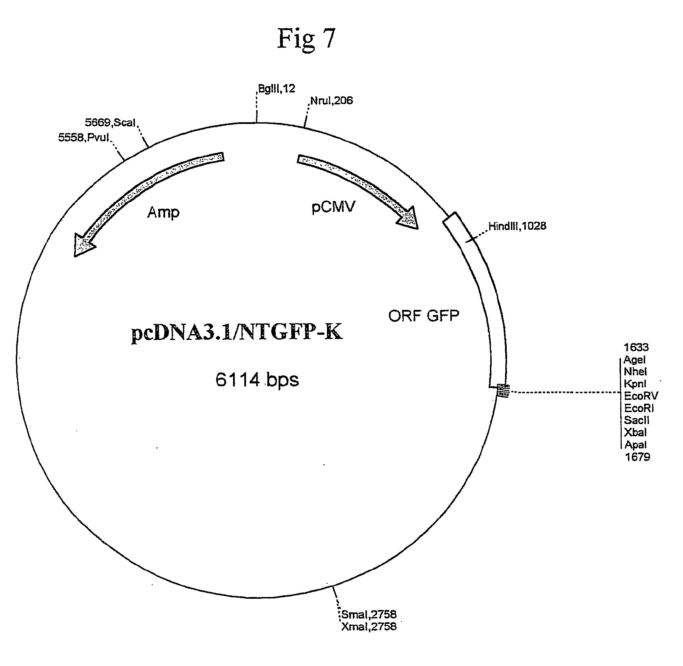

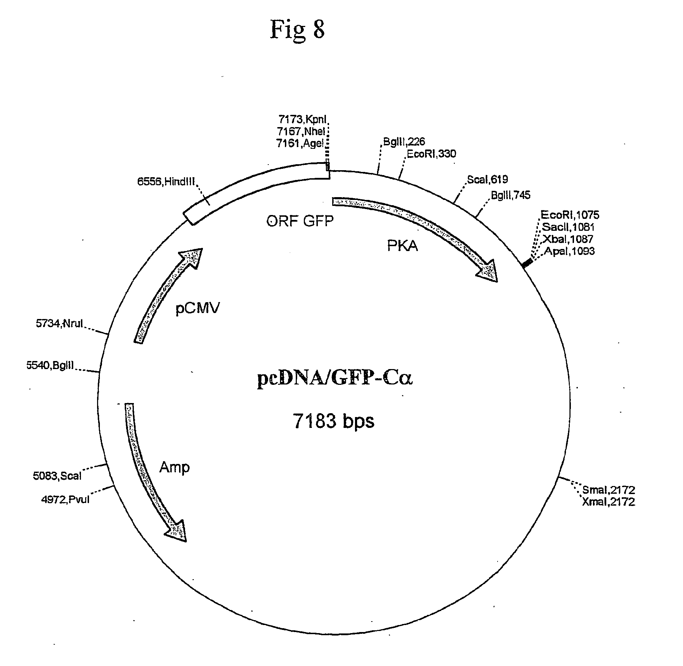

• Construction des vecteurs d'expression pcDNA3.1/RIIα (figure 4) et pcDNA3.1/RIIα-CAAX (figure 5) :
Le fragment de restriction SmaI-EcoRI codant pour les deux derniers acides aminés de la protéine RIIα et la séquence d'adressage à la membrane plasmique a été construit par hybridation de deux oligonuléotides sens et anti-sens (synthétisés et commercialisés par la société MWG BIOTECH France). Ce fragment de restriction a été ensuite cloné dans le vecteur pet11d/RIIα (Yuhan Wang et al., 1991, Proc. Natl. Acad. Sci. USA, 88, 2446-2450) ouvert par digestion par les enzymes de restriction SmaI et EcoRI pour donner le vecteur d'expression pet11d/RIIα-CAAX.
Les fragments de restriction NcoI-EcoRI contenant les séquences codant pour les protéines RIIα et RIIα-CAAX sont obtenus respectivement à partir des vecteurs d'expression pet11d/RIIα (figure 1) et pet11d/RIIα-CAAX (figure 2) par digestion par l'enzyme de restriction NcoI et traitement par l'enzyme de modification (fragment klenow de la polymérase I), suivi d'une digestion par l'enzyme de restriction EcoRI. Ces fragments sont ensuite sous-clonés dans le vecteur pcDNA3.1 (figure 3) (commercialisé sous la référence V790-20 par la société INVITROGEN LIFE TECHNOLOGIES, France) préalablement digéré par l'enzyme de restriction BamHI et traité par l'enzyme de modification (fragment klenow de la polymérase I), suivi d'une digestion par l'enzyme de restriction EcoRI pour donner les vecteurs d'expression pcDNA3.1/RIIα (figure 4) et pcDNA3.1/RIIα-CAAX (figure 5).
• Construction du vecteur d'expression pcDNA3.1/GFP-Cα (figure 8) :
Le fragment de restriction XhoI-HindIII correspondant au cadre de lecture de la sous-unité catalytique de la PKA est obtenu par digestion par les enzymes de restriction XhoI et HindIII et par traitement par l'enzyme de modification (fragment klenow de la polymérase I), à partir du vecteur d'expression pCMV-PKA (figure 6) (commercialisé sous la référence K6005-1 par la société CLONTECH LABORATORIES Inc., France). Ce fragment est ensuite sous-cloné dans le vecteur d'expression pcDNA3.1/NTGFP-K (figure 7) ouvert par digestion par l'enzyme de restriction EcoRV pour donner le vecteur d'expression pcDNA3.1/GFP-Cα (figure 8). Le vecteur pcDNA3.1/NTGFP-K correspond au vecteur pcDNA3.1/NT-GFP (commercialisé par la société INVITROGEN LIFE TECHNOLOGIES, France, sous la référence K4810-01), dans lequel a été substitué le site multiple de clonage.
EXEMPLE 2 : Obtention d'une cellule vivante exprimant les protéines codées par les constructions de l'exemple 1.
• Culture cellulaire et transfection :
La lignée cellulaire HEK 293 (« Human Embryonic Kidney » 293) a été établie à partir de cellules primaires embryonnaires de rein humain transformées par l'adénovirus de type 5. Ce sont des cellules adhérentes de type fibroblastique. Les HEK 293 sont maintenues en culture dans un milieu complet : le DMEM NUT MIX F12 (commercialisé sous la référence 31 331-028 par la société INVITROGEN LIFE TECHNOLOGIES, France) complémenté avec 10% de sérum de veau foetal (SVF), de la L-glutamine (2 mM), de la pénicilline (100 UI/ml) et de la streptomycine (100 µg/ml).
Les cellules HEK 293 cultivées à 80% de confluence sont transfectées avec les différentes constructions décrites ci-dessus en utilisant la Polyfect (QIAGEN, France, Réf 301107). L'ADN plasmidique est dilué dans du milieu DMEM NUT MIX F12 non complémenté. Après avoir additionné la Polyfect, ce mélange ADN/milieu/Polyfect est incubé 10 minutes à température ambiante. La proportion des différents agents en fonction de la surface des boites de culture est présentée au Tableau 3. Pendant ce temps d'incubation, le milieu de culture des cellules ensemencées la veille est remplacé par du milieu complet. Après les 10 minutes d'incubation, du milieu complet est ajouté au mélange ADN/milieu/Polyfect. Ce mélange de transfection est alors ajouté sur les cellules et homogénéisé. Les cellules sont incubées 24 heures à 37°C en présence de 5% de CO₂.

**TABLEAU 3**

| Type de boite de culture | Surface des boites (cm²) | DNA (µg) | Volume de milieu non complémenté (en µl) | Volume Polyfect (µl) | Volume de milieu complet à ajouter aux cellules (ml) | Volume de milieu complet à ajouter au mélange ADN/Polyfect (ml) |
|---|---|---|---|---|---|---|
| P35 Plaque 6 puits | 10 | 2 | 100 | 20 | 1,5 | 0,6 |
| P60 | 20 | 4 | 150 | 40 | 3,0 | 1 |
| P100 | 60 | 8 | 300 | 80 | 7,0 | 1 |

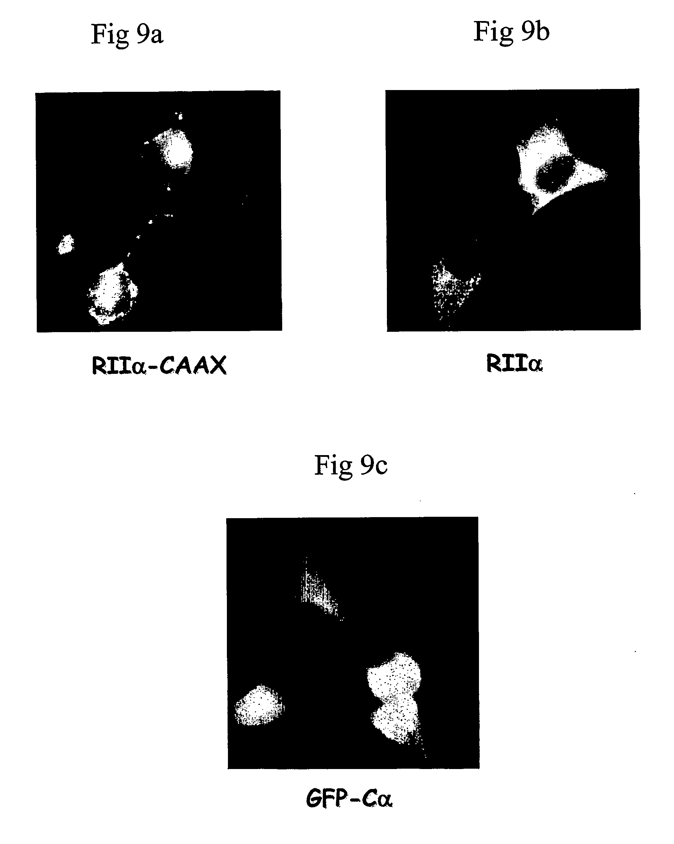

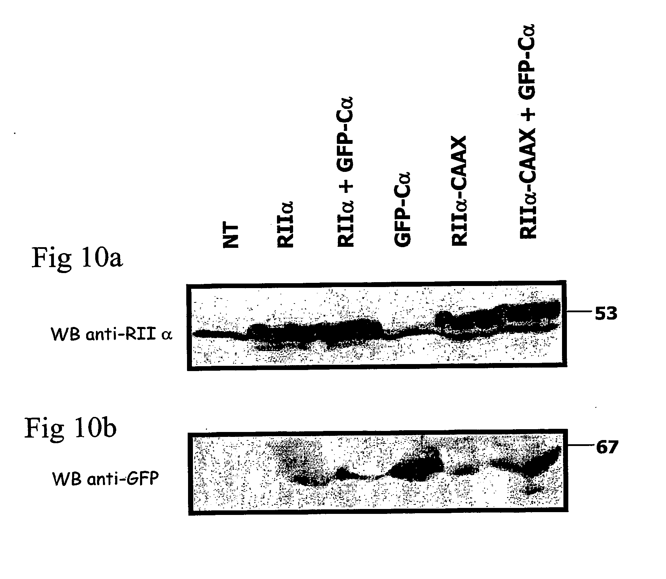

• Vérification de l'expression des protéines RIIα, RIIα-CAAX et GFP-Cα :
L'expression des protéines RIIα, RIIα-CAAX et GFP-Cα est vérifiée dans le système cellulaire HEK 293 par des techniques d'épifluorescence et de Western Blotting.
- Epifluorescence :
   Les cellules HEK 293 cultivées sur lamelles dans des boites de 10 cm² sont transfectées par le pcDNA3.1/RIIα, le pcDNA3.1/RIIα-CAAX ou le pcDNA3.1/GFP-Cα.
   Après 24 heures, les cellules transfectées avec le pcDNA3.1/RIIα ou le pcDNA3.1/RIIα-CAAX sont fixées 20 minutes à 4°C par la paraformaldéhyde 4%, puis perméabilisées par le méthanol 10 minutes à -20°C et par le triton X100 0,5% (commercialisé par la société EUROMEDEX, France) 5 minutes à température ambiante. Les cellules sont ensuite incubées 1 heure à température ambiante avec l'anticorps polyclonal anti-RIIα de lapin (commercialisé pr la société TEBU, FRANCE sous la référence PKA2areg(M-20):SC-909) à une dilution de 1/1000 dans du PBS (« Phosphate Buffer Saline », commercialisé par la société EUROMEDEX, France) complémenté avec 1% de SVF, puis incubées 45 minutes à température ambiante avec un anticorps couplé à une molécule fluorochrome et dirigé contre des Immunoglobulines G (IgG) de lapin à une dilution de 1/200 dans du PBS complémenté par 1% de SVF.
   Après 24 heures, les cellules transfectées par le pcDNA3.1/GFP-Cα sont fixées par la paraformaldéhyde 4%.
   Les cellules sont ensuite observées au moyen d'un microscope à fluorescence droit Axioplan équipé d'un objectif à immersion Gx40 et d'une caméra CCD. La longueur d'onde d'excitation est de 400 nm, la fluorescence est observée à 510 nm.
   Les cellules transfectées par le pcDNA3.1/RIIα-CAAX présentent un marquage membranaire (figure 9a) alors que le marquage observé sur les cellules transfectées avec le pcDNA3.1/RIIα, dépourvu de la séquence d'adressage à la membrane plasmique, reste exclusivement cytoplasmique (figure 9b). Les cellules transfectées par le pcDNA3.1/GFP-Cα montrent une fluorescence cytoplasmique et nucléaire (Figure 9c).
- Western Blotting (WB) :
   Des cellules HEK 293 cultivées dans des boites de culture de 60 cm² sont transfectées par le pcDNA3.1/RIIα, le pcDNA3.1/RIIα-CAAX ou le pcDNA3.1/GFP-Cα. 24 heures après, les cellules sont lavées deux fois avec 10 ml de PBS, puis reprises à 4°C dans un tampon de lyse complémenté en inhibiteurs de protéases et de phosphatases, dit LB+, contenant 50 mM Tris HCl pH 7,4 (INVITROGEN LIFE TECHNOLOGIES), 150 mM NaCl, 20 mM EDTA, 0,5% triton X100, 0,28 mM CHFO₂S, 1 mM NaVO₄, 10 µg/ml leupeptine, 10 µg/ml aprotinine (EUROMEDEX, France). Après une centrifugation (5 minutes à 17 800 g), la concentration en protéines du surnageant est estimée par la méthode de Bradford (selon le protocole proposé par la société BIO-RAD LABORATORIES, France) en référence à une courbe étalon de BSA (« Bovine Sérum Albumin »), décrite par la société ROCHE DIAGNOSTICS CORP, France. Les extraits protéiques totaux (100 µg), repris dans du LSB 1X (Laemmi Sample Buffer, Molecular Cloning : A Laboratory Manual, 2ème édition, volume 3, 1989), sont dénaturés 5 minutes à 95°C. Les échantillons sont déposés sur gel SDS-PAGE (« Sodium dodecyl sulfate, polyacrylamide gel electrophoresis ») à 8% d'acrylamide. Les protéines sont ensuite transférées sur une membrane de nitrocellulose, saturée par incubation à 4°C, toute la nuit, dans une solution de TBST (Tris Buffer Saline complémenté par 0,25% de Tween, commercialisé par la société EUROMEDEX, France) contenant 5% de lait (Régilait®, France). Les membranes sont ensuite lavées 3 fois 5 minutes dans du TBST, puis incubées 2 heures à température ambiante avec les anticorps primaires (anticorps anti-RIIα, dilution 1/500 ou anticorps polyclonal anti-GFP, dilution 1/400). Après 3 lavages de 5 minutes dans du TBST, les membranes sont incubées 45 minutes avec l'anticorps secondaire (anticorps anti-IgG de lapin couplé à la péroxydase du raifort, dilution 1/5000). L'hydrolyse de substrats par la péroxydase s'accompagne de la formation de composés luminescents détectables sur films photographiques selon le protocole ECL™ de la société AMERSHAM PHARMACIA BIOTECH, France.
   On observe une bande de 51 kDa reconnue par l'anticorps anti-RIIα dans les extraits protéiques obtenus à partir des cellules transfectées par le pcDNA3.1/RIIα ou le pcDNA3.1/RIIα-CAAX (figure 10a). Cette bande qui peut correspondre à RIIα ou RIIα-CAAX n'est pas détectée dans les extraits protéiques de cellules non transfectées (NT). De plus dans le cas de RIIα-CAAX, on détecte un retard de mobilité électrophorétique pouvant témoigner de la présence de la séquence d'adressage à la membrane plasmique (figure 10a). L'anticorps anti-RIIα reconnaît également une protéine de poids moléculaire apparent de 49 kDa dans les extraits protéiques de cellules transfectées ou non. Ceci peut s'expliquer par la présence dans les extraits de la sous-unité régulatrice RIIα endogène (figure 10a).
   On observe une bande de 60 kDa qui est reconnue par l'anticorps dirigé contre la GFP dans les extraits protéiques de cellules transfectées avec le vecteur pcDNA3.1/GFP-Cα. Cette bande qui correspond à GFP-Cα est absente dans les extraits protéiques obtenus à partir de cellules non transfectées (figure 10b).

• Co-localisation RIIα-CAAX et GFP-Cα :
Des expériences de co-localisation ont été réalisées afin d'apporter des arguments sur la formation d'un complexe protéique entre les sous-unités régulatrice et catalytique de la PKA à la membrane plasmique.
Des cellules sont cotransfectées avec le pcDNA3.1/RIIα et le pcDNA3.1/GFP-Cα ou avec le pcDNA3.1/RIIα-CAAX et le pcDNA3.1/GFP-Cα et observées au moyen d'un microscope à fluorescence droit.
Les résultats sont présentés dans le tableau 4.

**TABLEAU 4**

| Vecteurs transfectés | Immunofluorescence indirecte avec l'anticorps Anti -RIIα (1/1000) Observation filtre Rhodamine | Observation GFP-Cα Filtre FITC ou Filtre Ex:400nm Em:510nm |
|---|---|---|
| pcDNA3.1/RIIα+ pcDNA3.1/GFP-Cα | Marquage exclusivement cytoplasmique Co-localisation avec GFP-Cα | Marquage exclusivement cytoplasmique Co-localisation avec RIIα |
| pcDNA3.1 /RIIα-CAAX + pcDNA3.1/GFP-Cα | Marquage membranaire. Co-localisation avec GFP-Cα | Marquage membranaire. Co-localisation avec RIIα-CAAX |

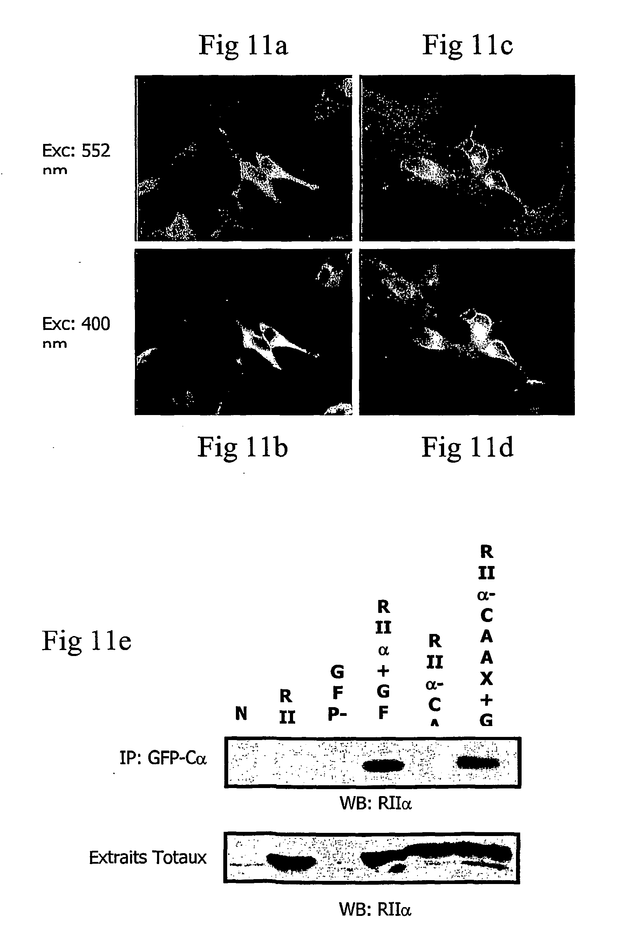

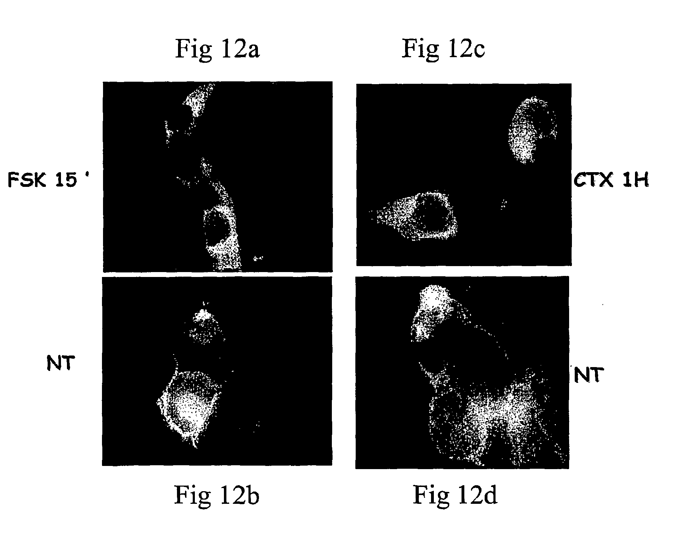

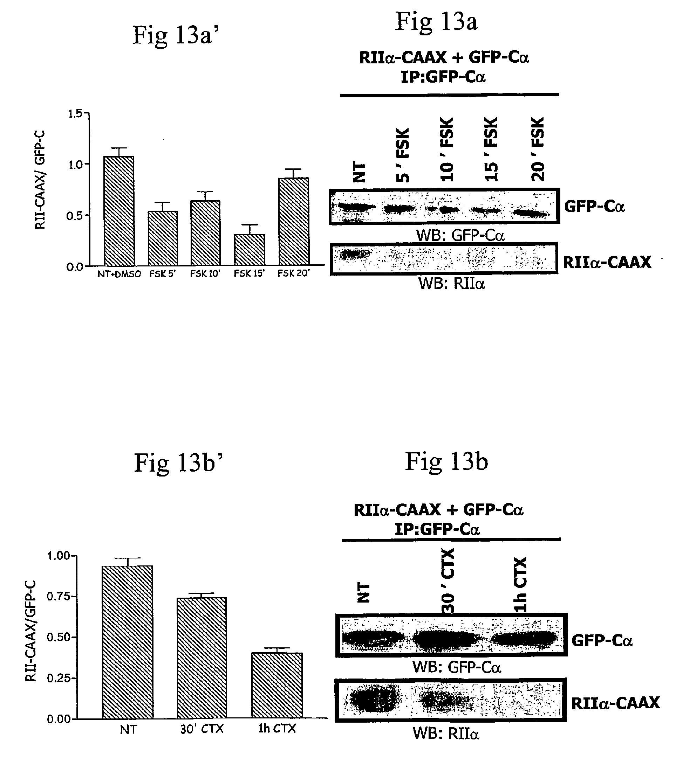

Les protéines RIIα, RIIα-CAAX, détectées avec l'anticorps dirigé contre RIIα, co-localisent bien avec la GFP-Cα (figures 11a, 11b, 11c et 11d). La localisation forcée à la membrane plasmique de la protéine RIIα-CAAX entraîne donc la relocalisation de la GFP-Cα au niveau de la membrane plasmique, cette relocalisation n'étant pas observée dans le cas où les cellules sont cotranfectées avec la RIIα dépourvue de la séquence d'adressage à la membrane plasmique.
• Interaction RIIα-CAAX /GFP-Cα :
Afin de confirmer l'interaction entre les protéines RIIα-CAAX et GFP-Cα, des expériences d'immunoprécipitation (IP) sont réalisées. Pour cela des extraits protéiques totaux sont préparés à partir de cellules HEK 293 transfectées avec le pcDNA3.1/RIIα, le pcDNA3.1/RIIα-CAAX ou le pcDNA3.1/GFP-Cα, ou à partir de cellules HEK 293 cotransfectées avec le pcDNA3.1/RIIα et le pcDNA3.1/GFP-Cα ou le pcDNA3.1/RIIα-CAAX et le pcDNA3.1/GFP-Cα. 1 mg d'extrait protéique est ensuite incubé dans 1 ml de LB+ en présence de 20 µl de billes A/G-Agarose couplées de façon covalente à l'anticorps monoclonal de souris dirigé contre la protéine GFP (ROCHE DIAGNOSTICS Corp, France, réf. 1814460) pendant 3 heures à 4°C sous agitation. Après trois lavages par 1 ml de LB+, les échantillons sont repris dans 50 µ1 de LSB 1X et déposés sur gel SDS-PAGE à 8% d'acrylamide et soumis à un Western blotting (WB) anti-RIIα.
Après immunoprécipitation avec l'anticorps dirigé contre la GFP, l'anticorps anti-RIIα reconnaît un bande de 51 kDa dans les extraits protéiques obtenus à partir des cellules exprimant les protéines RIIα et GFP-Cα ou à partir des cellules exprimant les protéines RIIα-CAAX et GFP-Cα (figure 11e). Cette bande de 51 kDa qui peut correspondre à RIIα ou RIIα-CAAX est absente dans les extraits protéiques de cellules non transfectées ou de cellules exprimant uniquement l'une des protéines (RIIα, RIIα-CAAX ou GFP-Cα).
Les protéines RIIα et RIIα-CAAX sont donc capables d'interagir avec la protéine GFP-Cα. Cependant cette expérience ne nous permet pas de déterminer si cette interaction est directe ou indirecte. Connaissant la structure de la PKA, il est tout à fait légitime de penser que cette interaction est directe. L'ajout de la séquence d'adressage à la membrane plasmique à la sous-unité régulatrice RIIα et la fusion de la GFP à la sous-unité catalytique Cα ne semble donc en aucun cas perturber la formation d'un complexe protéique entre la sous-unité régulatrice et les sous-unités catalytiques.
EXEMPLE 3 : Effet d'agents activant la voie de transduction de l'AMPc/PKA.
• Observation de l'activation de la voie AMPc/PKA sur des cellules HEK 293 fixées exprimant les protéines RIIα-CAAX et GFP-Cα :
Les résultats précédents montrent que les protéines RIIα-CAAX et GFP-Cα sont capables de s'associer pour former une PKA localisée à la membrane plasmique. Afin de déterminer si cette PKA est fonctionnelle, des expériences d'induction de la voie AMPc/PKA sont réalisées en traitant les cellules par la forskoline et la toxine du choléra. La forskoline agit directement sur l'adenylyl cyclase et entraîne son activation. La toxine du choléra catalyse l'ADP-ribosylation de la protéine Gs et conduit à son activation constitutive. Dans les deux cas le traitement conduit à l'augmentation du taux de synthèse de l'AMPc et par conséquent à l'augmentation du taux intracellulaire de l'AMPc. L'AMPc se lie sur les sous-unités régulatrices et entraîne de façon concomitante la dissociation des protéines GFP-Cα et leur libération dans le cytoplasme, évènements pouvant être suivis respectivement par détection en Western blotting de la sous-unité RIIα-CAAX après immunoprécipitation avec l'anticorps anti-GFP et par observation au microscope à fluorescence.
Les cellules HEK 293 sont cotransfectées avec les plasmides pcDNA3.1/RIIα-CAAX et pcDNA3.1/GFP-Cα. Après 24 heures d'incubation, le milieu de transfection est aspiré et remplacé par du DMEM NUT MIX F12 non complémenté contenant de la forskoline à une concentration finale de 10 µM ou de la toxine du choléra à 1µg/ml. Les cellules sont ensuite incubées à 37°C en présence de 5% de CO₂ pendant des temps variables. Après 2 lavages au PBS les cellules sont soit fixées par la paraformaldéhyde 4% puis observées au moyen d'un microscope à fluorescence, soit lysées pour obtenir des extraits protéiques afin de réaliser une immunoprécipitation avec l'anticorps anti-GFP suivi d'un Western blotting avec l'anticorps dirigé contre la protéine RIIα. Le rapport RIIα-CAAX/GFP-Cα est ensuite calculé en rapportant la quantité de protéines RIIα-CAAX détectées avec l'anticorps anti-RIIα après immunoprécipitation avec l'anticorps monoclonal anti-GFP à la quantité de protéines GFP-Cα immunoprécipitées dans l'extrait protéique total (détecté avec un anticorps polyclonal anti-GFP).
Les cellules exprimant RIIα-CAAX et GFP-Cα traitées à la forskoline (figure 12a) pendant 15 minutes ou à la toxine du choléra (figure 12c) pendant 1 heure présentent un marquage cytoplasmique alors que les cellules non traitées présentent un marquage membranaire (figure 12b et 12d). Par ailleurs, le rapport RIIα-CAAX/GFP-Cα met en évidence une réduction d'un facteur 3,5 de la quantité de protéines RIIα-CAAX qui co-immunoprécipitent avec les protéines GFP-Cα pour les cellules traitées à la forskoline pendant 15 minutes (figure 13a et 13a') par rapport aux cellules non traitées (NT). De la même façon, une réduction d'un facteur 2,5 de la quantité de protéines RIIα-CAAX qui co-immunoprécipitent avec les protéines GFP-Cα est observée pour les cellules traitées avec la toxine du choléra pendant 1 heure (figures 13b et 13b') par rapport aux cellules non traitées. Cette diminution du rapport RIIα-CAAX/GFP-Cα reflète la dissociation des sous-unités RIIα-CAAX et GFP-Cα en réponse à une augmentation du taux intracellulaire d'AMPc. L'augmentation du taux intracellulaire d'AMPc induite par les traitements à la forskoline ou à la toxine du choléra entraîne l'activation de la PKA localisée de manière constitutive à la membrane et par conséquent la dissociation et la libération dans le cytoplasme des sous-unités catalytiques fusionnées à la GFP.
• Observation de l'activation de la voie de l'AMPc/PKA dans des cellules COS-7 en culture exprimant les protéines RIIα-CAAX et GFP-Cα:
- Transfection :
   Pour cette expérience, on ensemence une Lab-Tek I à 2 compartiments (Nalge Nunc International, Naperville, USA, réf : 155380) avec 200 000 cellules COS-7 (ECACC n° 87021302), cultivées au 6ème passage. Le milieu de culture (1,5 ml/compartiment) est du DMEM (InvitrogenLifeTechnologies, Cergy-Pontoise, France, réf : 41966-029) complémenté avec 10% (v/v) de sérum de veau foetal (SVF), de la pénicilline à une concentration de 100 UI/ml et de la streptomycine à une concentration de 100 µg/ml. 24 heures après, les cellules sont cotransfectées avec les vecteurs d'expression pcDNA3.1/RIIα-CAAX et pcDNA3.1/GFP-Cα en utilisant la lipofectAMINE® 2000 (Invitrogen Life Technologies, réf :11668-019). 1,6 µg de pcDNA3.1/RIIα-CAAX plus 1,6 µg de pcDNA3.1/GFP-Cα, sont dilués dans 200 µl de DMEM non complémenté. Par ailleurs, on dilue 8 µl de lipofectAMINE® 2000 dans 200 µl de DMEM non complémenté et on incube ce mélange 5 minutes à température ambiante. On transfère ensuite la solution de lipofectAMINE® 2000 dans le mélange ADN/milieu puis on incube ce mélange 15 minutes à température ambiante. Pendant ce temps d'incubation, les cellules sont lavées deux fois avec 1 ml de DMEM non complémenté. On complète ensuite le mélange de transfection avec 1,6 ml de DMEM non complémenté. Après avoir homogénéisé, on ajoute le mélange de transfection complété avec le DMEM non complémenté (1 ml/compartiment) sur les cellules, et on incube les cellules pendant 4 heures à 37°C en présence de 5% de CO₂. On élimine le mélange de transfection par aspiration et on le remplace par 1,5 ml de DMEM complémenté avec 10% de SVF, de la pénicilline et de la streptomycine. Les cellules sont incubées 20 heures à 37°C en présence de 5% de CO₂.

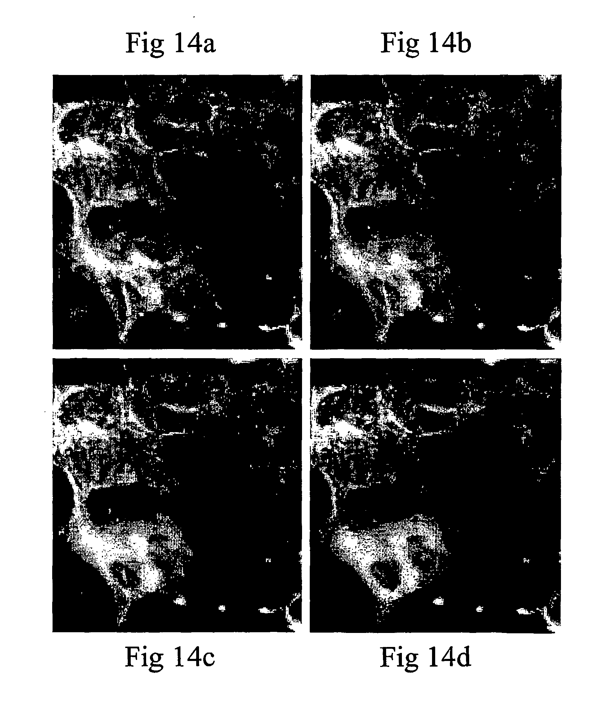

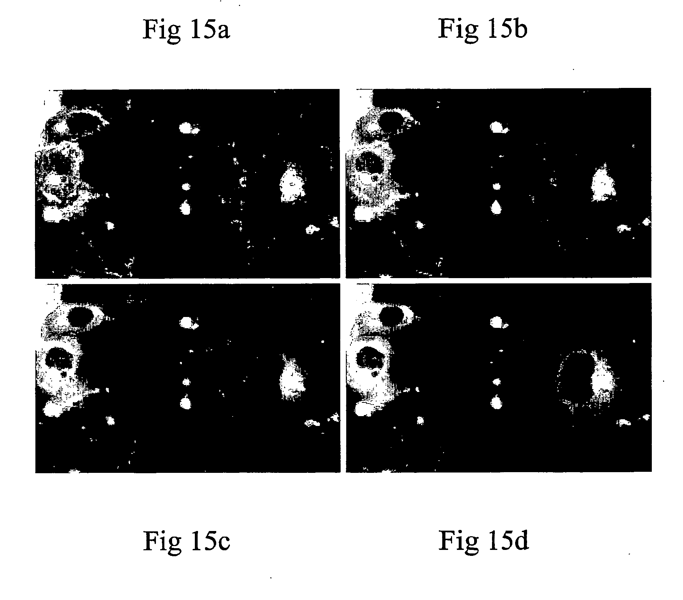

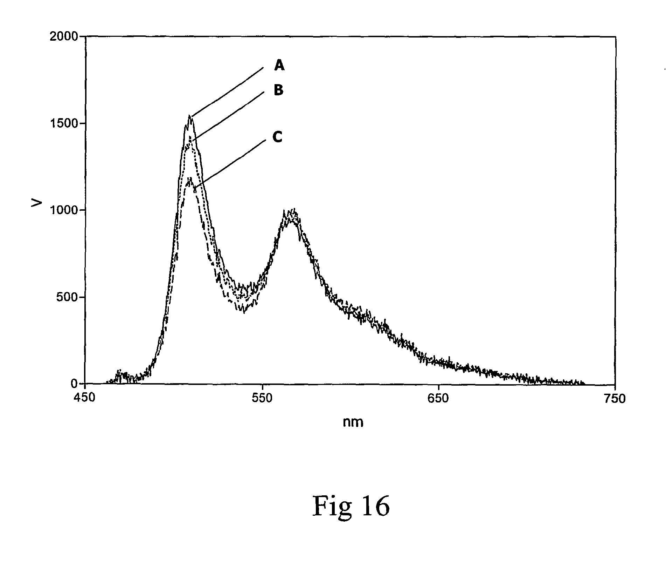

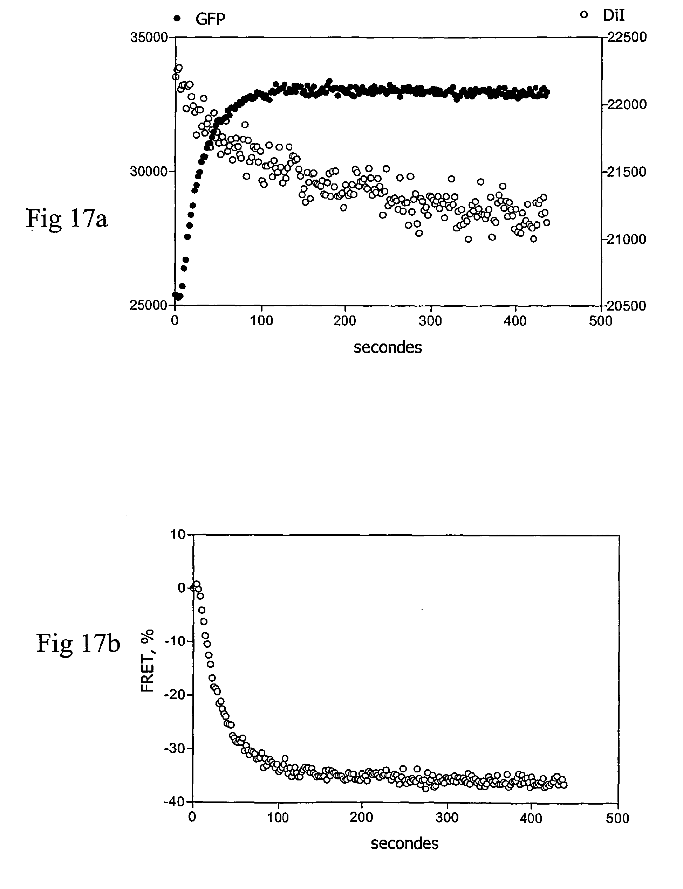

• Contrôle par imagerie de la translocation de la GFP-Cα de la membrane plasmique vers le cytoplasme lors d'un traitement avec la forskoline :
L'observation des cellules est réalisée avec le Vidéo Micro Spectro Fluorimètre : VMSF, il s'agit d'un microscope inversé (Leica DMIRB, Leica Microsystems, Rueil-Malmaison, France) couplé à un système d'acquisition d'image (Photométrics CoolSnapHQ™ piloté par MetaViewTM Imaging System, Roper Scientific Princeton Instruments, Evry, France) et à un spectrofluorimètre (Monochromateur : SpectraPro 150, détecteur : Spec-10 : 250 B piloté par WinSpec32, Roper Scientific™ Princeton Instruments). Cet outil répond aux exigences de travail sur des cellules vivantes et permet de visualiser, enregistrer et analyser dans le temps et dans l'espace des évènements cellulaires dynamiques.
Pour la visualisation au VMSF, le milieu de culture est aspiré et les cellules sont lavées 2 fois avec du PBS puis on ajoute le tampon Krebs Ringer, KRH (5mM KCl, 1,25 mM MgSO₄, 125 mM NaCl, 25 mM Hépes pH 7,4, 8 mM glucose, 1,5 mM CaCl₂, 0,1% BSA). Les cellules sont observées à l'objectif X20. La longueur d'onde d'excitation est de 400 nm (Filtre étroit réf : 99EFILT0265, filtre à l'excitation réf: 93/XF1008/N, Melles Griot Industrie, France), la lumière excitatrice est éliminée par réflexion en-deçà de 450 nm (filtre dichroïque 450DCLP, réf : 93/XF2006/N, Melles Griot Industrie), la fluorescence est observée à partir de 435 nm (filtre passe-haut 435ALP, réf : 93/XF3088/25R, Melles Griot Industrie). Au temps t = 0 le milieu est remplacé par du KRH contenant de la forskoline à une concentration finale de 20 µM, on acquiert ensuite une image toutes les secondes pendant 3 minutes. Certains des clichés sont représentés sur la figure 14. A t = 0 les cellules COS-7 présentent une fluorescence intense de type membranaire caractérisée par l'absence de contraste entre le cytoplasme et le noyau des cellules (figure 14a). Rapidement, on observe une perte de fluorescence au niveau des membranes plasmiques et une apparition concomitante de la fluorescence au niveau du cytoplasme caractérisé par un marquage diffus excluant la membrane et le noyau (figure 14b, 14c et 14d, respectivement avec t = 13s, t = 23s et t = 63s). A la différence des expériences réalisées sur des cellules fixées, on est capable d'observer un effet de la forskoline dès les premières secondes de traitement.
EXEMPLE 4: Effet d'agent médicamenteux (l'isoprotérénol) activant une cible thérapeutique (le récepteur β2 adrénergique) connue pour moduler la voie de transduction de l'AMPc/PKA.
Cet exemple a pour but d'étayer l'application du procédé au cribalge d'agents biologiquement actifs sur une cible thérapeutique donnée. L'isoprotérénol, appelé aussi isoprénaline, est un β31 et β2-mimétique commercialisé sous l'appelation de Isuprel®. Il a, outre son action sur les muscles lisses, un effet stimulant cardiaque et est indiqué dans le cas de bradycardie, de syndrome de Stokes-Adams (bloc auriculo-ventriculaire) et de choc à pression veineuse centrale élevée.
• Contrôle par imagerie de la translocation de la GFP-Cα de la membrane plasmique vers le cytoplasme lors d'un traitement avec l'isoprotérénol :
Des cellules COS-7 exprimant naturellement le récepteur β2 adrénergique, un récepteur couplé aux protéines G, sont cotransfectées avec les plasmides pcDNA3.1/RIIα-CAAX et pcDNA3.1/GFP-Cα. Après 24 heures d'incubation, le milieu de culture est aspiré et les cellules sont lavées 2 fois avec du PBS puis on ajoute le tampon KRH. Au temps t = 0 le milieu est remplacé par du KRH contenant de l'isoprotérénol à une concentration finale de 50 nM, on acquiert ensuite une image toutes les secondes pendant 3 minutes. Certains des clichés sont représentés sur la figure 15. A t = 0, les cellules exprimant RIIα-CAAX et GFP-Cα, présentent un marquage de type membranaire (figure 15a). Dès 5 secondes de traitement, on observe une diminution de la fluorescence au niveau de la membrane plasmique et une apparition concomitante de la fluorescence au niveau du cytoplasme (figure 15b). Après 10 secondes de traitement on discerne parfaitement, par contraste, les contours du noyau des cellules, caractéristique d'un marquage exclusivement cytoplasmique (figure 15c et 15d, respectivement avec t = 5s et t = 20s).
• Analyse par FRET de la translocation de la membrane plasmique vers le cytoplasme de GFP-Cα lors du traitement des cellules avec l'isoprotérénol :
La méthode choisie pour détecter et quantifier le changement d'état de la GFP-Cα (localisation membranaire versus localisation cytoplasmique) exploite le principe de FRET. Ce principe repose sur un processus physique, dépendant de la distance, par lequel l'énergie est transmise de manière non radiative d'un chromophore excité, le donneur, à un autre chromophore, l'accepteur, par couplage dipolaire. Des études préalables réalisées par les chercheurs ont permis d'établir un couple donneur/accepteur adapté à la mise en oeuvre du procédé où la GFP constitue le donneur et la sonde DiI l'accepteur (WO 03/005028). La sonde DiI est une molécule lipophile qui de part son affinité naturelle pour les membranes permet le marquage membranaire de cellules vivantes.
- Marquage avec la sonde DiI des membranes plasmiques de cellules COS-7 en culture cotransfectées avec les plasmides codant pour les protéines RIIα-CAAX et GFP-Cα :
   On ensemence une Lab-Tek I à 2 compartiments avec 200 000 cellules COS-7 (ECACC n° 87021302), cultivées au 5ème passage. 24 heures après, les cellules sont cotransfectées avec les plasmides pcDNA3.1/RIIα-CAAX et pcDNA3.1/GFP-Cα comme décrit dans l'exemple n° 3. 24 heures après la fin de la transfection les cellules sont marquées avec la sonde membranaire DiI. Pour ce faire, on prépare une solution de dioctadécyl-1,1'-tétraméthyl-3,3,3',3'-indocarbocyanine (DiI, Molecular Probes, Leiden, Pays Bas, réf: D-282) à une concentration de 10µg/µl dans la diméthylformamide. On prépare par ailleurs le milieu de marquage comprenant 1960 µl de milieu DMEM, 40 µl d'une solution PBS 5% BSA « fatty acid free » c'est à dire dépourvue d'acides gras (Sigma-Aldrich Chimie SARL, Saint-Quentin-Fallavier, France, réf: A-8806) chauffé à 37°C et dans lequel on disperse 10 µl de la solution de DiI à 10µg/ml. On obtient ainsi un milieu de marquage à une concentration finale en DiI de 50 µg/ml et contenant 0,1% de BSA « fatty acid free ». On aspire le milieu de culture du compartiment de la Lab-Tek I à 2 compartiments et on lave deux fois les cellules avec 1 ml de PBS. On ajoute ensuite 2 ml de milieu de marquage sur les cellules puis on les incube 10 minutes à 25°C. Après avoir aspiré le milieu de marquage, les cellules sont lavées trois fois avec du PBS. Pour finir on ajoute 1 ml de tampon KRH sur les cellules qui sont ensuite incubées 2 heures à 25°C.
- Analyse par spectrofluorimétrie de la fluorescence émise par des cellules COS-7 exprimant les protéines RIIα-CAAX et GFP-Cα et dont les membranes sont marquées avec la sonde DiI.
   Après 2 heures d'incubation à 25 °C la Lab-Tek I à 2 compartiments, contenant les cellules, est placé sur la platine chauffante du VMSF (température de travail 25°C). Après avoir aspiré le tampon KRH contenu dans le compartiment, les cellules sont observées à l'objectif X10 avec le même jeu de filtre utilisé dans l'exemple n° 3. La lumière collectée par l'objectif est transmise au spectrofluorimètre, on peut donc ainsi acquérir entre 450 nm et 700 nm le spectre d'émission de fluorescence des cellules exprimant les protéines RIIα-CAAX et GFP-Cα et marquées avec la sonde DiI. Les émissions de fluorescence de la GFP et du DiI se produisent respectivement avec un maximum à 510 nm et 565 nm. Au temps t = 0, on ajoute dans le compartiment 500 µl de tampon KRH contenant de l'isoprotérénol à une concentration finale de 50 nM et un inhibiteur de phosphodiestérases, le RO-20-1724, à une concentration finale de 100 µM. On enregistre alors 220 spectres à intervalle de 2 secondes. On enregistre ensuite un spectre d'émission de fluorescence d'une Lab-Tek I à 2 compartiments contenant un volume identique de KRH (soit dans ce cas 500 µl) que l'on soustrait à tous les spectres d'émission de fluorescence obtenus. On représente sur la figure 16 les spectres d'émission de fluorescence (Intensité de fluorescence, unité arbitraire (u.a) en fonction de la longueur d'onde, nanomètres (nm)) corrigés observés à t = 0 (spectre C), 26 (spectre B) et 438 secondes (spectre A). L'augmentation du taux intracellulaire d'AMPc induite par l'isoprotérénol entraîne la translocation de la GFP-Cα de la membrane plasmique vers le cytoplasme et donc une augmentation de la fluorescence de la GFP-Cα à 510 nm et une diminution simultanée de la fluorescence de la sonde DiI à 565 nm.
- Quantification de la variation de l'efficacité du transfert de fluorescence entre la GFP-Cα et la sonde DiI au cours de la translocation de la GFP-Cα dans le cytoplasme induite par l'isoprotérénol.
   L'efficacité du transfert non radiatif de fluorescence entre la GFP-Cα et la sonde DiI est donné comme le pourcentage de variation de fluorescence comparée à la fluorescence initiale : FRET % = 100 x (IO-It)/IO
   Où I0 est la fluorescence initiale mesurée sur des cellules non traitées et It est la fluorescence mesurée sur les cellules au temps t après traitement à l'isoprotérénol. En pratique, I0 et It sont calculés comme le rapport de la fluorescence de la GFP-Cα sur la fluorescence du DiI. La fluorescence de la GFP-Cα avant traitement ou au temps t après traitement est évaluée par intégration de son spectre de fluorescence corrigé entre 505 et 515 nm. La fluorescence du DiI avant traitement ou au temps t après traitement est évaluée par intégration de son spectre de fluorescence corrigé entre 560 et 570 nm. La figure 17a représente les variations de fluorescence de la GFP-Cα et du DiI au cours du temps à la suite du traitement à l'isoprotérénol. Après 100 secondes de traitement, on observe donc une augmentation de 27% de la fluorescence de la GFP-Cα et une diminution de 2,5% du DiI. Ceci se traduit par une perte de l'efficacité du transfert de la fluorescence de la GFP-Cα vers le DiI (figure 17b). Le FRET varie de 0% au moment de l'induction de la translocation de la protéine encore membranaire jusqu'à -39% après 200 secondes de traitement alors que la protéine est transloquée dans le cytoplasme.

## Revendications

1. Procédé de sélection d'agents, dits agents à tester, susceptibles d'être biologiquement actifs vis-à-vis d'un système cellulaire vivant en induisant ou en inhibant une synthèse intracellulaire d'AMPc lorsqu'ils sont en présence d'au moins une cellule sensible d'un système cellulaire vivant,
dans lequel :
- on prépare un système cellulaire à l'exception d'un embryon humain, d'un corps humain, d'un élément non isolé du corps humain ou d'une cellule souche embryonnaire humaine, comprenant au moins une cellule vivante sensible exprimant au moins une protéine recombinée multimérique spécifique de la voie de transduction AMPc/PKA,
• ladite protéine recombinée étant, en l'absence de synthèse induite d'AMPc, dans un premier état, dit état complexé, où elle forme un complexe, dit complexe RC, comprenant au moins une sous-unité régulatrice recombinée d'une PKA, dite unité R et comportant au moins un site de liaison de l'AMPc, et au moins une sous-unité catalytique recombinée d'une PKA, dite unité C,
• ladite protéine recombinée étant, en présence de synthèse induite d'AMPc, dans un deuxième état, dit état dissocié, où les unités R et C sont dissociées et où au moins une unité R fixe l'AMPc,
- on met en présence ledit système cellulaire avec une quantité d'au moins un agent à tester,
- on détecte l'état, complexé ou dissocié, de ladite protéine recombinée,
**caractérisé en ce que** :
- au moins l'une des unités R ou C, dite unité de translocation, présente une séquence protéique comprenant une séquence d'adressage membranaire présentant un motif de modification post-traductionnelle CAAX fonctionnel ; la position de ladite séquence d'adressage membranaire dans ladite séquence protéique étant adaptée pour préserver l'aptitude de la protéine recombinée à former à la membrane un complexe RC en l'absence de synthèse induite d'AMPc, et à se dissocier en présence de synthèse induite d'AMPc,
- au moins une autre unité R ou C, dite unité marquée, distincte d'une unité de translocation, présente un groupement, dit groupement de détection, adapté pour :
• préserver l'aptitude de la protéine recombinée à former un complexe RC en l'absence de synthèse induite d'AMPc, et à se dissocier en présence de synthèse induite d'AMPc, et
• permettre la détection de l'état de la protéine à l'aide d'un signal de détection qui, à la membrane, est différent selon l'absence ou la présence de synthèse induite d'AMPc.

2. Procédé de sélection selon la revendication 1, **caractérisé en ce que** le motif d'adressage membranaire de l'unité de translocation provient de l'extrémité C-terminale d'une protéine de type Ras.

3. Procédé de sélection selon l'une des revendications 1 ou 2, **caractérisé en ce que** le motif d'adressage comprend au moins une partie d'une séquence d'amino-acides choisie parmi : SEQ ID N°1, SEQ ID N°2, SEQ ID N°3 et SEQ ID N°4.

4. Procédé de sélection selon l'une des revendications 1 à 3, **caractérisé en ce que** le groupement de détection de l'unité marquée est un groupement luminophore choisi parmi :
- une protéine fluorescente naturelle ou une protéine fluorescente mutée, par addition, délétion ou substitution d'un ou plusieurs acides aminés d'une protéine naturelle, cette protéine fluorescente mutée conservant la propriété de fluorescence,
- un fragment fluorescent issu d'une protéine fluorescente naturelle ou d'une protéine fluorescence mutée adapté pour présenter la propriété de fluorescence de la protéine fluorescente dont il est issu,
- une protéine bioluminescente naturelle ou mutée, par addition, délétion ou substitution d'un ou plusieurs acides aminés d'une protéine naturelle, cette protéine bioluminescente mutée conservation la propriété de bioluminescence,
- la fluorescéine, la rhodamine, la Bodipy®, la cyanine, le Nile Red®, Texas Red®, les chélates de terres rares, les indo- et carbocyamines, le diphénylhexatriène les dérivés aromatiques luminescents.

5. Procédé de sélection selon les revendications 1 à 4, **caractérisé en ce que** l'unité de translocation est l'unité R et l'unité marquée est l'unité C.

6. Procédé de sélection selon l'une des revendications 1 à 5, **caractérisé en ce que** l'état, complexé ou dissocié, de la protéine recombinée multimérique spécifique de la voie de transduction AMPc/PKA se fait par la détection, respectivement, d'une émission ou d'une extinction lumineuse du couplage de luminescence entre deux groupements luminescents complémentaires, en exploitant le principe de FRET ou BRET.

7. Procédé de sélection selon la revendication 6, **caractérisé en ce qu'**on introduit dans le système cellulaire, une composition d'au moins un composé, dit agent membranaire de couplage, apte à s'associer à la membrane plasmique de cellule sensible et marqué avec au moins un groupement photocomplémentaire, dit deuxième luminophore, distinct du groupement luminescent porté par l'unité marquée, dit premier luminophore complémentaire du deuxième luminophore, et de telle sorte que les propriétés du système cellulaire vivant vis-à-vis du signal cellulaire soient au moins sensiblement préservées, et qu'au moins une propriété mesurable de l'émission lumineuse entre le premier et le deuxième luminophores soit sensiblement modifée lorsque le signal cellulaire change d'état.

8. Procédé de sélection selon la revendication 7, **caractérisé en ce que** l'un au moins des premier et deuxième luminophores est choisi parmi :
- une molécule susceptible d'absorber la lumière émise par une protéine fluorescente,
- une protéine fluorescente naturelle ou une protéine fluorescente mutée, par addition, délétion ou substitution d'un ou plusieurs acides aminés d'une protéine fluorescente naturelle, cette protéine fluorescente mutée conservant la propriété de fluorescence,
- un fragment fluorescent issu d'une protéine fluorescente naturelle ou d'une protéine fluorescente mutée, adapté pour présenter la propriété de fluorescence de la protéine fluorescente dont il est issu,
- une protéine bioluminescente naturelle -notamment de type luciférase- ou une protéine bioluminescente mutée, par addition, délétion ou substitution d'un ou plusieurs acides aminés d'une protéine naturelle, cette protéine bioluminescente mutée conservant la propriété de bioluminescence,
- la fluorescéine, la rhodamine, la Bodipy®, le Nile Red@, le Texas Red®, les chélates de terres rares, les indo- et carbocyanines, le diphénylhexatriène, les dérivés aromatiques luminescents.

9. Procédé de sélection selon l'une des revendications 7 ou 8, **caractérisé en ce que** l'un au moins des premier et deuxième luminophores est une protéine autofluorescente naturelle de cnidaire.

10. Procédé de sélection selon l'une des revendications 7 à 9, **caractérisé en ce qu'**on utilise un agent membranaire de couplage comprenant un ligand lipophile choisi parmi les phospholipides, les acides gras, les glycérides, le cholestérol, les céramides, les amines grasses, les agents luminophores lipophiles ou les dérivés lipophiles de ces composés.

11. Procédé de sélection selon l'une des revendications 1 à 10, **caractérisé en ce que** l'unité de translocation est une unité R issue de la fusion par voie génétique :
- d'au moins une partie de la séquence protéique d'une sous-unité régulatrice choisie parmi les isoformes : RIα, RIβ, RIIα, RIIβ.
- et d'une séquence d'adressage membranaire.

12. Procédé de sélection selon la revendication 11, **caractérisé en ce que** l'unité de translocation a pour séquence protéique SEQ ID N°5.

13. Procédé de sélection selon les revendications 11 ou 12, **caractérisé en ce que** l'unité de translocation est codée par la séquence nucléique SEQ ID N°6.

14. Procédé de sélection selon l'une des revendications 1 à 13, **caractérisé en ce que** l'unité marquée est une séquence protéique comprenant au moins une partie de la séquence d'une sous-unité catalytique choisie parmi les isoformes : Cα, Cβ et Cγ,
marquée par un groupement de détection.

15. Système cellulaire vivant à l'exception d'un embryon humain, d'un corps humain, d'un élément non isolé du corps humain ou d'une cellule souche embryonnaire humaine, contenant au moins une cellule vivante exprimant au moins une protéine recombinée multimérique spécifique de la voie de transduction AMPc/PKA,
- ladite protéine recombinée étant, en l'absence de synthèse induite d'AMPc, dans un premier état, dit état complexé, où elle forme un complexe, dit complexe RC, comprenant au moins une sous-unité régulatrice recombinée d'une PKA, dite unité R et comportant au moins un site de liaison de l'AMPc, et au moins une sous-unité catalytique recombinée d'une PKA, dite unité C,
- ladite protéine recombinée étant, en présence de synthèse induite d'AMPc, dans un deuxième état, dit état dissocié, où les unités R et C sont dissociées et où au moins une unité R fixe l'AMPc,
**caractérisé en ce que** :
- au moins l'une des unités R ou C, dite unité de translocation, présente une séquence protéique comprenant une séquence d'adressage membranaire présentant un motif de modification post-traductionnelle CAAX fonctionnel ; la position de ladite séquence d'adressage membranaire dans ladite séquence protéique étant adaptée pour préserver l'aptitude de la protéine recombinée à former à la membrane un complexe RC en l'absence de synthèse induite d'AMPc, et à se dissocier en présence de synthèse induite d'AMPc,
- au moins une autre unité R ou C, dite unité marquée, distincte d'une unité de translocation, présente un groupement, dit groupement de détection, adapté pour :
• préserver l'aptitude de la protéine recombinée à former un complexe RC en l'absence de synthèse induite d'AMPc, et à se dissocier en présence de synthèse induite d'AMPc, et
• permettre la détection de l'état de la protéine à l'aide d'un signal de détection qui, à la membrane, est différent selon l'absence ou la présence de synthèse induite d'AMPc.

16. Système cellulaire selon la revendication 15, **caractérisé en ce que** le motif d'adressage membranaire de l'unité de translocation provient de l'extrémité C-terminale d'une protéine de type Ras.

17. Système cellulaire selon l'une des revendications 15 ou 16, **caractérisé en ce que** le motif d'adressage comprend au moins une partie d'une séquence d'amino-acides choisie parmi : SEQ ID N°1, SEQ ID N°2, SEQ ID N°3 et SEQ ID N°4.

18. Système cellulaire selon l'une des revendications 15 à 17, **caractérisé en ce que** le groupement de détection de l'unité marquée est un groupement luminophore choisi parmi :
- une protéine fluorescente naturelle ou une protéine fluorescente mutée, par addition, délétion ou substitution d'un ou plusieurs acides aminés d'une protéine naturelle, cette protéine fluorescente mutée conservant la propriété de fluorescence,
- un fragment fluorescent issu d'une protéine fluorescente naturelle ou d'une protéine fluorescente mutée, adapté pour présenter la propriété de fluorescence de la protéine fluorescente dont il est issu,
- une protéine bioluminescente naturelle -notamment de type luciférase- ou une protéine bioluminescente mutée, par addition, délétion ou substitution d'un ou plusieurs acides aminés d'une protéine naturelle, cette protéine bioluminescente mutée conservant la propriété de bioluminescence,
- la fluorescéine, la rhodamine, le Bodipy®, la cyanine, le Nile Red@, Texas Red@, les chélates de terres rares, les indo- et carbocyamines, le diphénylhexatriène les dérivés aromatiques luminescents.

19. Système cellulaire selon les revendications 15 à 18, **caractérisé en ce que** l'unité de translocation est l'unité R et l'unité marquée est l'unité C.

20. Système de sélection selon l'une des revendications 15 à 19, **caractérisé en ce que** l'état, complexé ou dissocié, de la protéine recombinée multimérique spécifique de la voie de transduction AMPc/PKA se fait par la détection, respectivement, d'une émission ou d'une extinction lumineuse du couplage de luminescence entre deux groupements luminescents complémentaires, en exploitant le principe de FRET ou BRET.

21. Système cellulaire selon la revendication 20, **caractérisé en ce qu'**il comprend au moins un composé, dit agent membranaire de couplage, associé à la membrane plasmique et marqué avec au moins un groupement photocomplémentaire, dit deuxième luminophore, distinct du groupement luminescent porté par l'unité marquée, dit premier luminophore complémentaire du deuxième luminophore, et de telle sorte que les propriétés du système cellulaire vivant vis-à-vis du signal cellulaire soient au moins sensiblement préservées, et qu'au moins une propriété mesurable de l'émission lumineuse entre le premier et le deuxième luminophores soit sensiblement modifiée lorsque le signal cellulaire change d'état.

22. Système cellulaire selon la revendication 21, **caractérisé en ce que** l'un au moins des premier et deuxième luminophores est choisi parmi :
- une molécule susceptible d'absorber la lumière émise par une protéine fluorescente,
- une protéine fluorescente naturelle ou une protéine fluorescente mutée, par addition, délétion ou substitution d'un ou plusieurs acides aminés d'une protéine fluorescente naturelle, cette protéine fluorescente mutée conservant la propriété de fluorescence,
- un fragment fluorescent issu d'une protéine fluorescente naturelle ou d'une protéine fluorescente mutée, adapté pour présenter la propriété de fluorescence de la protéine fluorescente dont il est issu,
- une protéine bioluminescente naturelle ou une protéine bioluminescente mutée, par addition, délétion ou substitution d'un ou plusieurs acides aminés d'une protéine naturelle, cette protéine bioluminescente mutée conservant la propriété de bioluminescence,
- la fluorescéine, la rhodamine, le Bodipy®, le Nile Red@, le Texas Red®, les chélates de terres rares, les indo- et carbocyanines, le diphénylhexatriène, les dérivés aromatiques luminescents.

23. Système cellulaire selon l'une des revendications 21 ou 22, **caractérisé en ce que** l'un au moins des premier et deuxième luminophores est une protéine autofluorescente naturelle de cnidaire.

24. Système cellulaire selon l'une des revendications 20 à 23, **caractérisé en ce qu'**on utilise un agent membranaire de couplage comprenant un ligand lipophile choisi parmi les phospholipides, les acides gras, les glycérides, le cholestérol, les céramides, les amines grasses, les agents luminophores lipophiles ou les dérivés lipophiles de ces composés.

25. Système cellulaire selon l'une des revendications 15 à 24, **caractérisé en ce que** l'unité de translocation est une sous-unité régulatrice recombinée issue de la fusion par voie génétique :
- d'au moins une partie de la séquence protéique d'une sous-unité régulatrice choisie parmi les isoformes : RIα, RIβ, RIIα et RIIβ,
- et d'une séquence d'adressage membranaire.

26. Système cellulaire selon la revendication 25, **caractérisé en ce que** l'unité de translocation à pour séquence protéique SEQ ID N°5.

27. Système cellulaire selon l'une des revendications 25 ou 26, **caractérisé en ce que** l'unité de translocation est codée par la séquence nucléique SEQ ID N°6.

28. Système cellulaire selon l'une des revendications 15 à 27, **caractérisé en ce que** l'unité marquée est une séquence protéique comprenant au moins une partie de la séquence d'une sous-unité catalytique choisie parmi les isoformes : Cα, Cβ et Cγ,
marquée d'un groupement de détection.

29. Protéine recombinée susceptible d'être exprimée au niveau de la membrane plasmique d'une cellule, de fixer l'AMPc, de se lier à une sous-unité catalytique de la PKA lorsqu'elle ne fixe par d'AMPc et de se dissocier de ladite sous-unité catalytique lorsqu'elle fixe l'AMPc, comprenant une séquence protéique choisie parmi la séquence protéique SEQ ID N°5, la séquence protéique SEQ ID N°5 clivée de ses résidus V-L-S carboxyl-terminaux.

30. Séquence nucléique codant pour une protéine recombinée selon la revendication 29, comprenant une séquence nucléique SEQ ID N°6.

## Claims

1. A method for the selection of agents, designated agents to be tested, capable of being biologically active towards a living cellular system by inducing or inhibiting intracellular cAMP synthesis when said agents are in the presence of at least one sensitive cell of a living cellular system, in which:
- a cellular system, with the exception of a human embryo, a human body, a non-isolated element of the human body or a human embryonic stem cell, is prepared comprising at least one sensitive living cell which expresses at least one specific multimeric recombinant protein of the cAMP/PKA transduction pathway,
• said recombinant protein being, in the absence of induced cAMP synthesis, in a first state, designated complexed state, in which it forms a complex, designated RC complex, comprising at least one recombinant regulatory subunit of a PKA, designated R unit, and comprising at least one cAMP binding site, and at least one recombinant catalytic subunit of a PKA, designated C unit,
• said recombinant protein being, in the presence of induced cAMP synthesis, in a second state, designated dissociated state, in which the R and C units are dissociated and in which at least one R unit fixes cAMP,
- said cellular system is brought into contact with a quantity of at least one agent to be tested,
- the state, complexed or dissociated, of said recombinant protein is detected,
**characterised in that**:
- at least one of the R or C units, designated translocation unit, has a protein sequence comprising a membrane addressing sequence having a functional post-translational modification CAAX motif; the position of said membrane addressing sequence in said protein sequence being capable of preserving the ability of the recombinant protein to form an RC complex on the membrane in the absence of induced cAMP synthesis and to dissociate in the presence of induced cAMP synthesis,
- at least one other R or C unit, designated labelled unit, distinct from a translocation unit, has a group, designated detection group, capable of:
• preserving the ability of the recombinant protein to form an RC complex in the absence of induced cAMP synthesis, and to dissociate in the presence of induced cAMP synthesis, and
• permitting detection of the state of the protein with the assistance of a detection signal which, on the membrane, differs depending on the absence or the presence of induced cAMP synthesis.

2. A selection method according to claim 1, **characterised in that** the membrane addressing motif of the translocation unit originates from the C-terminal end of a Ras type protein.

3. A selection method according to either of claim 1 or claim 2, **characterised in that** the addressing motif comprises at least part of an amino acid sequence selected from among: SEQ ID No. 1, SEQ ID No. 2, SEQ ID No. 3 and SEQ ID No. 4.

4. A selection method according to any one of claims 1 to 3, **characterised in that** the detection group of the labelled unit is a luminophore group selected from among:
- a natural fluorescent protein or a mutant fluorescent protein, formed by addition, deletion or substitution of one or more amino acids of a natural protein, said mutant fluorescent protein retaining the property of fluorescence,
- a fluorescent fragment originating from a natural fluorescent protein or a mutant fluorescent protein capable of exhibiting the property of fluorescence of the fluorescent protein from which it originates,
- a natural or mutant bioluminescent protein, formed by addition, deletion or substitution of one or more amino acids of a natural protein, said mutant bioluminescent protein retaining the property of bioluminescence,
- fluorescein, rhodamine, Bodipy®, cyanine, Nile Red®, Texas Red@, rare earth chelates, indo- and carbocyanines, diphenylhexatriene, luminescent aromatic derivatives.

5. A selection method according to claims 1 to 4, **characterised in that** the translocation unit is the R unit and the labelled unit is the C unit.

6. A selection method according to any one of claims 1 to 5, **characterised in that** the state, complexed or dissociated, of the specific multimeric recombinant protein of the cAMP/PKA transduction pathway is detected by detection, respectively, of light emission or extinction from the luminescence coupling between two complementary luminescent groups by exploiting the FRET or BRET principle.

7. A selection method according to claim 6, **characterised in that** a composition is introduced into the cellular system, said composition comprising at least one compound, designated membrane coupling agent, capable of associating with the sensitive cell plasmic membrane and labelled with at least one photocomplementary group, designated second luminophore, distinct from the luminescent group borne by the labelled unit, designated first luminophore complementary to the second luminophore, and in such manner that the properties of the living cellular system with regard to the cellular signal are at least substantially preserved, and **in that** at least one measurable property of the light emission between the first and the second luminophores is substantially modified when the cellular signal changes state.

8. A selection method according to claim 7, **characterised in that** at least one of the first and second luminophores is selected from among:
- a molecule capable of absorbing the light emitted by a fluorescent protein,
- a natural fluorescent protein or a mutant fluorescent protein, formed by addition, deletion or substitution of one or more amino acids of a natural fluorescent protein, said mutant fluorescent protein retaining the property of fluorescence,
- a fluorescent fragment originating from a natural fluorescent protein or a mutant fluorescent protein capable of exhibiting the property of fluorescence of the fluorescent protein from which it originates,
- a natural bioluminescent protein, in particular of the luciferase type, or a mutant bioluminescent protein, formed by addition, deletion or substitution of one or more amino acids of a natural protein, said mutant bioluminescent protein retaining the property of bioluminescence,
- fluorescein, rhodamine, Bodipy®, Nile Red®, Texas Red®, rare earth chelates, indo- and carbocyanines, diphenylhexatriene, luminescent aromatic derivatives.

9. A selection method according to either of claim 7 or claim 8, **characterised in that** at least one of the first and second luminophores is a natural cnidarian autofluorescent protein.

10. A selection method according to any one of claims 7 to 9, **characterised in that** a membrane coupling agent is used which comprises a lipophilic ligand selected from among phospholipids, fatty acids, glycerides, cholesterol, ceramides, fatty amines, lipophilic luminophoric agents or the lipophilic derivatives of these compounds.

11. A selection method according to any one of claims 1 to 10, **characterised in that** the translocation unit is an R unit originating from the genetic fusion:
- of at least part of the protein sequence of a regulatory subunit selected from among the isoforms: RIα, RIβ, RIIα, RIIβ.
- and a membrane addressing sequence.

12. A selection method according to claim 11, **characterised in that** the translocation unit has SEQ ID No. 5 as the protein sequence.

13. A selection method according to claim 11 or claim 12, **characterised in that** the translocation unit is encoded by the nucleic sequence SEQ ID No. 6.

14. A selection method according to any one of claims 1 to 13, **characterised in that** the labelled unit is a protein sequence comprising at least part of the sequence of a catalytic subunit selected from among the isoforms: Cα, Cβ and Cγ, labelled by a detection group.

15. A living cellular system, with the exception of a human embryo, a human body, a non-isolated element of the human body or a human embryonic stem cell, containing at least one living cell which expresses at least one specific multimeric recombinant protein of the cAMP/PKA transduction pathway,
- said recombinant protein being, in the absence of induced cAMP synthesis, in a first state, designated complexed state, in which it forms a complex, designated RC complex, comprising at least one recombinant regulatory subunit of a PKA, designated R unit, and comprising at least one cAMP binding site, and at least one recombinant catalytic subunit of a PKA, designated C unit,
- said recombinant protein being, in the presence of induced cAMP synthesis, in a second state, designated dissociated state, in which the R and C units are dissociated and in which at least one R unit fixes cAMP,
**characterised in that**:
- at least one of the R or C units, designated translocation unit, has a protein sequence comprising a membrane addressing sequence having a functional post-translational modification CAAX motif; the position of said membrane addressing sequence in said protein sequence being capable of preserving the ability of the recombinant protein to form an RC complex on the membrane in the absence of induced cAMP synthesis and to dissociate in the presence of induced cAMP synthesis,
- at least one other R or C unit, designated labelled unit, distinct from a translocation unit, has a group, designated detection group, capable of:
• preserving the ability of the recombinant protein to form an RC complex in the absence of induced cAMP synthesis, and to dissociate in the presence of induced cAMP synthesis, and
• permitting detection of the state of the protein with the assistance of a detection signal which, on the membrane, differs depending on the absence or the presence of induced cAMP synthesis.

16. A cellular system according to claim 15, **characterised in that** the membrane addressing motif of the translocation unit originates from the C-terminal end of a Ras type protein.

17. A cellular system according to either of claim 15 or claim 16, **characterised in that** the addressing motif comprises at least part of an amino acid sequence selected from among: SEQ ID No. 1, SEQ ID No. 2, SEQ ID No. 3 and SEQ ID No. 4.

18. A cellular system according to any one of claims 15 to 17, **characterised in that** the detection group of the labelled unit is a luminophore group selected from among:
- a natural fluorescent protein or a mutant fluorescent protein, formed by addition, deletion or substitution of one or more amino acids of a natural protein, said mutant fluorescent protein retaining the property of fluorescence,
- a fluorescent fragment originating from a natural fluorescent protein or a mutant fluorescent protein capable of exhibiting the property of fluorescence of the fluorescent protein from which it originates,
- a natural bioluminescent protein, in particular of the luciferase type, or a mutant bioluminescent protein, formed by addition, deletion or substitution of one or more amino acids of a natural protein, said mutant bioluminescent protein retaining the property of bioluminescence,
- fluorescein, rhodamine, Bodipy®, cyanine, Nile Red®, Texas Red®, rare earth chelates, indo- and carbocyanines, diphenylhexatriene, luminescent aromatic derivatives.

19. A cellular system according to claims 15 to 18, **characterised in that** the translocation unit is the R unit and labelled unit is the C unit.

20. A cellular system according to any one of claims 15 to 19, **characterised in that** the state, complexed or dissociated, of the specific multimeric recombinant protein of the cAMP/PKA transduction pathway is detected by detection, respectively, of light emission or extinction from the luminescence coupling between two complementary luminescent groups by exploiting the FRET or BRET principle.

21. A cellular system according to claim 20, **characterised in that** it comprises at least one compound, designated membrane coupling agent, associated with the plasmic membrane and labelled with at least one photocomplementary group, designated second luminophore, distinct from the luminescent group borne by the labelled unit, designated first luminophore complementary to the second luminophore, and in such manner that the properties of the living cellular system with regard to the cellular signal are at least substantially preserved, and **in that** at least one measurable property of the light emission between the first and the second luminophores is substantially modified when the cellular signal changes state.

22. A cellular system according to claim 21, **characterised in that** at least one of the first and second luminophores is selected from among:
- a molecule capable of absorbing the light emitted by a fluorescent protein,
- a natural fluorescent protein or a mutant fluorescent protein, formed by addition, deletion or substitution of one or more amino acids of a natural fluorescent protein, said mutant fluorescent protein retaining the property of fluorescence,
- a fluorescent fragment originating from a natural fluorescent protein or a mutant fluorescent protein capable of exhibiting the property of fluorescence of the fluorescent protein from which it originates,
- a natural bioluminescent protein or a mutant bioluminescent protein, formed by addition, deletion or substitution of one or more amino acids of a natural protein, said mutant bioluminescent protein retaining the property of bioluminescence,
- fluorescein, rhodamine, Bodipy®, Nile Red®, Texas Red®, rare earth chelates, indo- and carbocyanines, diphenylhexatriene, luminescent aromatic derivatives.

23. A cellular system according to either of claim 21 or claim 22, **characterised in that** at least one of the first and second luminophores is a natural cnidarian autofluorescent protein.

24. A cellular system according to any one of claims 20 to 23, **characterised in that** a membrane coupling agent is used which comprises a lipophilic ligand selected from among phospholipids, fatty acids, glycerides, cholesterol, ceramides, fatty amines, lipophilic luminophoric agents or the lipophilic derivatives of these compounds.

25. A cellular system according to any one of claims 15 to 24, **characterised in that** the translocation unit is a recombinant regulatory subunit originating from the genetic fusion:
- of at least part of the protein sequence of a regulatory subunit selected from among the isoforms: RIα, RIβ, RIIα and RIIβ,
- and a membrane addressing sequence.

26. A cellular system according to claim 25, **characterised in that** the translocation unit has SEQ ID No. 5 as the protein sequence.

27. A cellular system according to either of claim 25 or claim 26, **characterised in that** the translocation unit is encoded by the nucleic sequence SEQ ID No. 6.

28. A cellular system according to any one of claims 15 to 27, **characterised in that** the labelled unit is a protein sequence comprising at least part of the sequence of a catalytic subunit selected from among the isoforms: Cα, Cβ and Cγ, labelled by a detection group.

29. A recombinant protein capable of being expressed at the plasmic membrane of a cell, of fixing cAMP, of binding to a catalytic subunit of PKA when it does not fix cAMP and of dissociating from said catalytic subunit when it does fix cAMP, comprising a protein sequence selected from among protein sequence SEQ ID No. 5, protein sequence SEQ ID No. 5 cleaved from its carboxy-terminal V-L-S residues.

30. A nucleic sequence encoding a recombinant protein according to claim 29, comprising a nucleic sequence SEQ ID No. 6.

## Patentansprüche

1. Auswahlverfahren von Wirkstoffen, bezeichnet als zu testende Wirkstoffe, die geeignet sind, gegenüber einem lebenden Zellsystem biologisch aktiv zu sein, indem sie eine intrazelluläre AMPc-Synthese induzieren oder inhibieren, wenn sie sich in Gegenwart von wenigstens einer sensiblen Zelle eines lebenden Zellsystems befinden, in dem:
- ein Zellsystem - mit Ausnahme eines menschlichen Embryos, eines menschlichen Körpers, eines nicht isolierten Elements des menschlichen Körpers, einer Stammzelle eines menschlichen Embryos - zubereitet wird, das wenigstens eine sensible lebende Zelle umfasst, die wenigstens ein rekombinantes, multimerisches, spezifisches Protein des Transduktionweges AMPc/PKA exprimiert,
* wobei das genannte rekombinante Protein bei Fehlen der induzierten AMPc-Synthese in einem ersten, als komplexer Zustand bezeichneten Zustand ist, in dem sie einen als RC-Komplex bezeichneten Komplex bildet, wenigstens eine rekombinante regulierende Untereinheit einer PKA, bezeichnet als Einheit R, umfasst, und wenigstens einen Verbindungssitus des AMPc, und wenigstens eine rekombinante, katalytische Untereinheit PKA, bezeichnet als Einheit C,
* wobei das genannte rekombinante Protein bei Vorhandensein einer induzierten AMPc-Synthese in einem zweiten Zustand ist, bezeichnet als dissoziierter Zustand, in dem die Einheiten R und C dissoziiert sind und in dem wenigstens eine Einheit R am AMPc fixiert ist;
- das genannte Zellsystem mit einer Menge von wenigstens einem zu testenden Wirkstoff in Kontakt gebracht wird,
- der dissoziierte oder komplexierte Zustand des genannten rekombinanten Proteins detektiert wird,
**dadurch gekennzeichnet, dass**:
- wenigstens eine der Einheiten R oder C, bezeichnet als Translokation, eine proteinartige Sequenz aufweist, die eine Membranadressierungssequenz umfasst, die ein funktionales Modifikationsmotiv CAAX nach der Übersetzung aufweist; wobei die Position der genannten Membranadressierungssequenz in der genannten proteinartigen Sequenz angepasst ist, um bei Fehlen der induzierten AMPc-Synthese die Fähigkeit des rekombinanten Proteins zur Bildung eines Komplexes RC an der Membran zu bewahren und sich bei Vorhandensein der induzierten AMPc-Synthese zu dissoziieren,
- wenigstens eine andere Einheit R oder C, bezeichnet als markiert, die von einer Translokationseinheit unterschiedlich ist, eine Gruppierung aufweist, bezeichnet als Detektionsgruppierung, die angepasst ist, um:
* die Fähigkeit des rekombinanten Proteins zur Bildung eines Komplexes RC bei Fehlen der induzierten AMPc-Synthese zu bewahren und sich bei Vorhandensein der induzierten AMPc-Synthese zu dissoziieren, und
* die Detektion des Zustandes des Proteins mithilfe eines Detektionssignals zu erlauben, das an der Membran je nach Fehlen oder Vorhandensein der induzierten AMPc-Synthese unterschiedlich ist.

2. Auswahlverfahren gemäß Anspruch 1, **dadurch gekennzeichnet, dass** das Membranadressierungsverfahren der Translokationseinheit aus dem Ende mit C-Endung eines Proteins vom Typ Ras stammt.

3. Auswahlverfahren gemäß Anspruch 1 oder 2, **dadurch gekennzeichnet, dass** das Adressierungsmotiv wenigstens einen Teil einer Aminosäuresequenz umfasst, die ausgewählt ist aus: SEQ ID NR. 1, SEQ ID NR. 2, SEQ ID NR. 3 und SEQ ID NR. 4.

4. Auswahlverfahren gemäß Anspruch 1 bis 3, **dadurch gekennzeichnet, dass** die Detektionsgruppierung der markierten Einheit eine Luminophorgruppierung ist, die ausgewählt ist aus:
- einem natürlichen fluoreszierenden Proteins oder einem per Addition, Deletion oder Substitution einer oder mehrerer Aminosäuren eines natürlichen Proteins mutierten fluoreszierenden Proteins, wobei dieses mutierte fluoreszierende Protein die fluoreszierende Eigenschaft beibehält,
- einem fluoreszierenden Fragment, das aus einem natürlichen fluoreszierenden Protein oder einem mutierten fluoreszierenden Protein stammt, das angepasst ist, um die Fluoreszenzeigenschaft des fluoreszierenden Proteins aufzuweisen, aus dem es stammt,
- einem natürlichen oder per Addition, Deletion oder Substitution einer oder mehrerer Aminosäuren eines natürlichen Proteins mutierten biolumineszierenden Proteins, wobei dieses mutierte lumineszierende Protein die Biolumineszenzeigenschaft beibehält,
- Fluroreszein, Rhodamin, Bodipy®, Zyanin, Nile Red®, Texas Red@, Chelate seltener Erden, Indo- und Carbozyanine, Diphenylhexatrien, die Derivate aromatischer Lumineszenzen.

5. Auswahlverfahren gemäß Anspruch 1 bis 4, **dadurch gekennzeichnet, dass** die Translokationseinheit die Einheit R ist und die markierte Einheit die Einheit C ist.

6. Auswahlverfahren gemäß Anspruch 1 bis 5, **dadurch gekennzeichnet, dass** der komplexierte oder dissoziierte Zustand des rekombinanten multimerischen spezifischen Proteins des Transduktionsweges AMPc/PKA durch die Detektion jeweils einer Lichtausgabe oder eines Lichtlöschens der Lumineszenzkopplung zwischen zwei komplementären lumineszierenden Gruppierungen detektiert wird, indem das Prinzip FRET oder BRET genutzt wird.

7. Auswahlverfahren gemäß Anspruch 6, **dadurch gekennzeichnet, dass** in das Zellsystem eine Zusammensetzung aus wenigstens einer Komponente, bezeichnet als Kopplungsmembranwirkstoff, eingeführt wird, die geeignet ist, sich mit der plasmischen Membran der sensiblen Zelle zu verbinden und mit wenigstens einer fotokomplementären Gruppierung, bezeichnet als zweiter Luminophor, markiert ist, der von der lumineszierenden Gruppierung unterschiedlich ist, die von der markierten Einheit getragen wird, bezeichnet als erster komplementärer Luminophor des zweiten Luminophors, und derart, dass die Eigenschaften des lebenden Zellsystems gegenüber dem Zellsignal wenigstens deutlich gewahrt bleiben, und dass wenigstens eine messbare Eigenschaft der Lichtausgabe zwischen dem ersten und dem zweiten Luminophor deutlich modifiziert wird, wenn das Zellsignal den Zustand ändert.

8. Auswahlverfahren gemäß Anspruch 7, **dadurch gekennzeichnet, dass** wenigstens der erste und der zweite Luminophor ausgewählt wird aus:
- einem Molekül, das geeignet ist, das von einem fluoreszierenden Protein ausgegebene Licht zu absorbieren,
- einem natürlichen fluoreszierenden Protein oder einem per Addition, Deletion oder Substitution einer oder mehrerer Aminosäure(n) eines natürlichen fluoreszierenden Proteins mutierten fluoreszierenden Protein, wobei dieses mutierte fluoreszierende Protein die Fluoreszenzeigenschaft beibehält,
- einem fluoreszierenden Fragment, das aus einem fluoreszierenden natürlichen Protein oder einem fluoreszierenden mutierten Protein stammt und angepasst ist, um die Fluoreszenzeigenschaft des fluoreszierenden Proteins zu bewahren, aus dem es stammt,
- einem natürlichen biolumineszierenden Protein - insbesondere vom Typ Luciferase - oder einem per Addition, Deletion oder Substitution einer oder mehrerer Aminosäure(n) eines natürlichen Proteins mutierten biolumineszierenden Protein, wobei dieses mutierte biolumineszierende Protein die Biolumineszenzeigenschaft beibehält,
- Fluoreszein, Rhodamin, Bodipy®, Nile Red@, Texas Rad®, Chelate seltener Erden, Indo- und Carbocyanine, Diphenylhexatrien, aromatische lumineszierende Derivate.

9. Auswahlverfahren gemäß Anspruch 7 oder 8, **dadurch gekennzeichnet, dass** wenigstens der erste und der zweite Luminophor ein natürliches autofluoreszierendes Protein aus Nesseltierchen ist.

10. Auswahlverfahren gemäß Anspruch 7 bis 9, **dadurch gekennzeichnet, dass** ein Kopplungsmembranwirkstoff verwendet wird, der einen lipophilen Liganden umfasst, der aus Phospholipiden, Fettsäuren, Glyceriden, Cholesterin, Ceramiden, fetten Aminen, lipophilen luminophoren Wirkstoffen oder lipophilen Derivaten dieser Komponenten ausgewählt ist.

11. Auswahlverfahren gemäß Anspruch 1 bis 10, **dadurch gekennzeichnet, dass** die Translokationseinheit eine Einheit R ist, die aus der Fusion auf genetischem Wege stammt:
- wenigstens eines Teils der proteinartigen Sequenz einer regulierenden Untereinheit, die aus den folgenden Isoformen ausgewählt ist: RIα, RIß, RIIα, RIIß.
- und einer Membranadressierungssequenz.

12. Auswahlverfahren gemäß Anspruch 11, **dadurch gekennzeichnet, dass** die Translokationseinheit SEQ ID Nr. 5 als proteinartige Sequenz hat.

13. Auswahlverfahren gemäß Anspruch 11 oder 12, **dadurch gekennzeichnet, dass** die Translokationseinheit durch die Nukleinsequenz SEQ ID Nr. 6 kodiert ist.

14. Auswahlverfahren gemäß Anspruch 1 bis 13, **dadurch gekennzeichnet, dass** die markierte Einheit eine proteinartige Sequenz ist, die wenigstens einen Teil der Sequenz einer katalytischen Untereinheit umfasst, die aus den Isoformen: Cα, Cβ und Cγ ausgewählt ist, und durch eine Detektionsgruppe markiert ist.

15. Lebendes Zellsystem, mit Ausnahme eines menschlichen Embryos, eines menschlichen Körpers, eines nicht isolierten Elements des menschlichen Körpers oder einer Stammzelle eines menschlichen Embryos, das wenigstens eine lebende Zelle enthält, die wenigstens ein spezifisches, multimerischen, rekombinantes Protein des Transduktionsweges AMPc/PKA enthält,
- wobei das genannte rekombinante Protein bei Fehlen der induzierten AMPc-Synthese in einem ersten Zustand ist, bezeichnet als komplexierter Zustand, in dem sie einen Komplex bildet, bezeichnet als Komplex RC, der wenigstens eine regulierende rekombinante Untereinheit einer PKA umfasst, bezeichnet als Einheit R, und wenigstens einen Verbindungssitus des AMPc umfasst und wenigstens eine katalytische rekombinante Untereinheit einer PKA, bezeichnet als Einheit C,
- wobei das genannte rekombinante Protein bei Vorhandensein der induzierten AMPc-Synthese in einem zweiten Zustand ist, bezeichnet als dissoziierter Zustand, in dem die Einheiten R und C dissoziiert sind und in dem wenigstens eine Einheit R das AMPc fixiert,
**dadurch gekennzeichnet, dass**:
- wenigstens eine der Einheiten R oder C, bezeichnet als Translokation, eine proteinartige Sequenz aufweist, die eine Membranadressierungssequenz umfasst, die ein funktionales Modifikationsmotiv CAAX nach der Übersetzung aufweist; wobei die Position der genannten Membranadressierungssequenz in der genannten proteinartigen Sequenz angepasst ist, um die Fähigkeit des rekombinanten Proteins zur Bildung eines Komplexes RC an der Membran bei Fehlen der induzierten AMPc-Synthese zu bewahren und sich bei Vorhandensein der induzierten Synthese AMPc zu dissoziieren,
- wenigstens eine andere Einheit R oder C, bezeichnet als markierte Einheit, die unterschiedlich von einer Translokationseinheit ist, eine Gruppierung aufweist, bezeichnet als Detektionsgruppierung, die angepasst ist, um:
- die Fähigkeit des rekombinanten Proteins zur Bildung eines Komplexes RC bei Fehlen der induzierten AMPc-Synthese zu bewahren und sich bei Vorhandensein der induzierten AMPc-Synthese zu dissoziieren, und
* die Detektion des Zustandes des Proteins mithilfe eines Detektionssignals zu erlauben, das an der Membran je nach dem Fehlen oder dem Vorhandensein der induzierten AMPc-Synthese unterschiedlich ist.

16. Zellsystem gemäß Anspruch 15, **dadurch gekennzeichnet, dass** das Membranadressiermotiv der Translokationseinheit aus dem Ende mit C-Endung eines Proteins vom Typ Ras stammt.

17. Zellsystem gemäß Anspruch 15 oder 16, **dadurch gekennzeichnet, dass** das Adressiermotiv wenigstens einen Teil einer Aminosäuresequenz umfasst, die ausgewählt ist aus: SEQ ID NR. 1, SEQ ID NR. 2, SEQ ID NR. 3 und SEQ ID NR. 4.

18. Zellsystem gemäß Anspruch 15 bis 17, **dadurch gekennzeichnet, dass** die Detektionsgruppierung der markierten Einheit eine luminophore Gruppierung ist, die ausgewählt ist aus:
- einem natürlichen fluoreszierenden Protein oder einem per Addition, Deletion oder Substitution einer oder mehrerer Aminosäure(n) eines natürlichen Proteins mutierten fluoreszierenden Protein, wobei dieses mutierte fluoreszierende Protein die Fluoreszenzeigenschaft beibehält,
- einem fluoreszierenden Fragment, das aus einem natürlichen fluoreszierenden Protein oder einem mutierten fluoreszierenden Protein stammt, das angepasst ist, um die Fluoreszenzeigenschaft des fluoreszierenden Proteins zu bewahren, aus dem es stammt.
- einem natürlichen biolumineszierendes Protein - insbesondere vom Typ Luciferase - oder einem durch Addition, Deletion oder Substitution einer oder mehrerer Aminosäure(n) eines natürlichen Proteins mutierten Biolumineszenzprotein, wobei dieses mutierte biolumineszierende Protein die Biolumineszenzeigenschaft beibehält,
- Fluoreszein, Rhodamin, Bodipy®, Zyanin, Nile Red®, Texas Red@, Chelate seltener Erden, Indo- und Carbozyanine, Diphenylhexatrien, die aromatischen lumineszierenden Derivate.

19. Zellsystem gemäß Anspruch 15 bis 18, **dadurch gekennzeichnet, dass** die Translokationseinheit die Einheit R ist und die markierte Einheit die Einheit C ist.

20. Zellsystem gemäß Anspruch 15 bis 19, **dadurch gekennzeichnet, dass** der komplexierte oder dissoziierte Zustand des rekombinanten multimerischen spezifischen Proteins des Transduktionsweges AMPc/PKA durch Detektion jeweils einer Lichtausgabe oder eines Lichtlöschens der Lumineszenzkopplung zwischen zwei komplementieren lumineszierenden Gruppierungen unter Ausnutzen des Prinzips FRET oder BRET detektiert wird.

21. Zellsystem gemäß Anspruch 20, **dadurch gekennzeichnet, dass** es wenigstens eine Komponente umfasst, bezeichnet als Membrankopplung, die mit der plasmischen Membran verbunden ist und mit wenigstens einer fotokomplementären Gruppierung, bezeichnet als zweiter Luminophor, markiert ist, die unterschiedlich von der lumineszierenden Gruppierung ist, die von der markierten Einheit getragen wird, bezeichnet als erster komplementärer Luminophor des zweiten Luminophors, derart, dass die Eigenschaften des lebenden Zellsystem gegenüber dem Zellsignal wenigstens deutlich bewahrt bleiben, und dass wenigstens eine messbare Eigenschaft der Lichtausgabe zwischen dem ersten und dem zweiten Luminophor deutlich modifiziert wird, wenn das Zellsignal den Zustand ändert.

22. Zellsystem gemäß Anspruch 21, **dadurch gekennzeichnet, dass** wenigstens der erste und der zweite Luminophor ausgewählt ist aus:
- einem Molekül, das geeignet ist, das von einem fluoreszierenden Protein ausgegebene Licht zu absorbieren,
- einem natürlichen fluoreszierenden Protein oder einem per Addition, Deletion oder Substitution einer oder mehrerer Aminosäure(n) eines natürlichen fluoreszierenden Proteins mutierten fluoreszierenden Protein, wobei dieses mutierte fluoreszierende Protein die Fluoreszenzeigenschaft bewahrt.
- einem fluoreszierenden Fragment, das aus einem natürlichen fluoreszierenden Protein oder einem mutierten fluoreszierenden Protein stammt, das angepasst ist, um die Fluoreszenzeigenschaft des fluoreszierenden Proteins zu bewahren, aus dem es stammt,
- einem natürlichen biolumineszierenden Protein oder einem per Addition, Deletion oder Substitution einer oder mehrerer Aminosäure(n) eines natürlichen Proteins mutierten biolumineszierenden Protein, wobei dieses mutierte biolumineszierende Protein die Biolumineszenzeigenschaft beibehält,
- Fluoreszein, Rhodamin, Bodipy®, Nile Red@, Texas Red®, Chelate seltener Erden, Indo- und Carbozyanine, Diphenylhexatrien, den aromatischen lumineszierenden Derivaten.

23. Zellsystem gemäß Anspruch 21 oder 22, **dadurch gekennzeichnet, dass** wenigstens einer des ersten und des zweiten Luminophors ein natürliches autofluoreszierendes Protein aus Nesseltierchen ist.

24. Zellsystem gemäß Anspruch 20 bis 23, **dadurch gekennzeichnet, dass** ein Kopplungsmembranwirkstoff verwendet wird, der einen lipophilen Liganden umfasst, der aus Phospholipiden, Fettsäuren, Glyceriden, Cholesterin, Ceramiden, fetten Aminen, lipophilen luminophoren Wirkstoffen oder den lipophilen Derivaten dieser Komponenten ausgewählt sind.

25. Zellsystem gemäß Anspruch 15 bis 24, **dadurch gekennzeichnet, dass** die Translokationseinheit eine rekombinante regulierende Untereinheit ist, die aus der Fusion per genetischem Wege stammt:
- aus wenigstens einem Teil der proteinartigen Sequenz einer regulierenden Untereinheit, die ausgewählt ist aus den Isoformen: RIα, RIß, RIIα und RIIß,
und einer Membranadressierungssequenz.

26. Zellsystem gemäß Anspruch 25, **dadurch gekennzeichnet, dass** die Translokationseinheit SEQ ID NR. 5 als proteinartige Sequenz hat.

27. Zellsystem gemäß Anspruch 25 oder 26, **dadurch gekennzeichnet, dass** die Translokationseinheit durch die Nukleinsequenz SEQ ID NR. 6 kodiert ist.

28. Zellsystem gemäß Anspruch 15 bis 27, **dadurch gekennzeichnet, dass** die markierte Einheit eine proteinartige Sequenz ist, die wenigstens einen Teil der Sequenz einer katalytischen Untereinheit umfasst, die ausgewählt ist aus den Isoformen: Cα, Cβ und Cγ,
und mit einer Detektionsgruppierung markiert ist.

29. Rekombinantes Protein, das geeignet ist, bei der plasmischen Membran einer Zelle ausgedrückt zu werden, das AMPc zu fixieren, sich mit einer katalytischen Untereinheit der PKA zu verbinden, wenn sie nicht durch AMPc fixiert ist und sich von der genannten katalytischen Untereinheit zu dissoziieren, wenn sie das AMPc fixiert, umfassend eine proteinartige Sequenz, die ausgewählt ist aus der proteinartigen Sequenz SEQ ID NR. 5, der proteinartigen Sequenz ID NR. 5, die aus ihren Rückständen V-L-S Carboxyl-Endungen abgespalten ist.

30. Für ein rekombinantes Protein kodierende Nukleinsequenz gemäß Anspruch 29, die eine Nukleinsequenz SEQ ID NR. 6 umfasst.


### LISTAGE DE SEQUENCES

<110> NOVALEADS
<120> Procédé de sélection d'agents bio-actifs par détection d'une variation induite de la concentration intracellulaire d'AMPc d'une cellule vivante sensible.
<130> N01550-BE10426
<160> 10
<170> PatentIn version 3.1
<210> 1
   <211> 20
   <212> PRT
   <213> Homo sapiens
<400>- 1
<210> 2
   <211> 20
   <212> PRT
   <213> Homo sapiens
<400> 2
   Lys Ile Ser Lys Glu Glu Lys Thr Pro Gly Cys Val Lys Ile Lys Lys
   1               5                   10                  15
   Cys Ile Ile Met
               20
<210> 3
   <211> 19
   <212> PRT
   <213> Homo sapiens
<400> 3
   Met Ser Lys Asp Gly Lys Lys Lys Lys Lys Lys Ser Lys Thr Lys Cys
   1               5                   10                  15
   Val Ile Met
<210> 4
   <211> 20
   <212> PRT
   <213> Homo sapiens
<400> 4
   Lys Leu Asn Ser Ser Asp Asp Gly Thr Gln Gly Cys Met Gly Leu Pro
   1               5                   10                  15
   Cys Val Val Met
               20
<210> 5
   <211> 421
   <212> PRT
   <213> Artificial Sequence
   <220>
   <223> Hybrid protein synthetised by the genetic fusion between RIIa and the CAAX containing domain of H-Ras.
<400> 5
<210> 6
   <211> 1267
   <212> DNA
   <213> Artificial Sequence
   <220>
   <223> Nucleic acid encoding for the hybrid protein RIIa-CAAX.
<400> 6
<210> 7
   <211> 17
   <212> PRT
   <213> Varied species
<400> 7
   Gly Ser Phe Gly Glu Leu Ala Leu Ile Tyr Gly Thr Pro Arg Ala Ala
   1               5                   10                  15
   Thr
<210> 8
   <211> 17
   <212> PRT
   <213> Varied species
<400> 8
   Gly Ser Phe Gly Glu Leu Ala Leu Met Tyr Gly Thr Pro Arg Ala Ala
   1               5                   10                  15
   Thr
<210> 9
   <211> 17
   <212> PRT
   <213> Varied species
<400> 9
   Gly Ser Phe Gly Glu Leu Ala Leu Ile Tyr Asn Thr Pro Arg Ala Ala
   1               5                   10                  15
   Thr
<210> 10
   <211> 17
   <212> PRT
   <213> Varied species
<400> 10
   Gly Ser Phe Gly Glu Leu Ala Leu Ile Tyr Asn Thr Pro Arg Ala Ala
   1               5                   10                  15
   Thr

### LISTAGE DE SEQUENCES

<11Θ> NOVALEADS
<12Θ> Procédé de sélection d'agents bio-actifs par détection d'une variation induite de la concentration intracellulaire d'AMPc d'une cellule vivante sensible.
<13Θ> N01550-BE10426
<16Θ> 10
<17Θ> PatentIn version 3.1
<21Θ> 1
   <211> 20
   <212> PRT
   <213> Homo sapiens
<400> 1
<21Θ> 2
   <211> 2Θ
   <212> PRT
   <213> Homo sapiens
<4ΘΘ> 2
<21Θ> 3
   <211> 19
   <212> PRT
   <213> Homo sapiens
<400> 3